# EUROPEAN PATENT SPECIFICATION

(11) **EP 1 707 187 B1**
(45) Date of publication and mention of the grant of the patent: **01.08.2007**
(21) Application number: 05007229.7
(22) Date of filing: 02.04.2005
(51) Int. Cl.: A61K 8/49, A61Q 5/10

(54) **Reductive colorant for keratin fibres**
System zum reduktiven Färben von Keratinfasern
Système colorant reductif pour fibres de keratine

(43) Date of publication of application: 04.10.2006
(73) Proprietor: Wella Aktiengesellschaft, 64274 Darmstadt (DE)
(72) Inventor: Speckbacher, Markus, 3175 Flamatt (CH); Braun, Hans-Jürgen, 3182 Ueberstorf (CH)

(56) References cited:
- EP-A- 0 873 744
- WO-A-00/33799
- WO-A-01/13882
- WO-A-20/04022016
- US-A- 3 337 411

## Description

The present invention relates to a reductive colouring system consisting of oxime compounds and carbonyl compounds, and ascorbic acid as reducing agent, and to colorants comprising these compounds for keratin fibres, such as, for example, human hair, wool or furs.

In general, two processes are used for colouring keratin-containing fibres. One possibility consists in the use of direct dyes. These are incorporated into suitable carrier masses in order then to be applied to the fibres. This method, generally known as tinting, is easy to use, exceptionally mild and is characterized by low damage to the keratin fibres since no ammonia or peroxide is added. However, the durability and wash resistance of this colouring method is generally unsatisfactory, for which reason a direct method is also referred to as semipermanent hair colour.

A long-lasting coloration, also called permanent hair colour, can be produced with oxidation dyes which are formed by oxidative coupling of one or more developer components with one or more coupler components. If required, oxidation-stable direct dyes can also be added to the oxidative system in order to achieve particular colour effects. When using oxidizing agents, however, damage to the hair structure is observed. Furthermore, some of the oxidation hair colour precursors used (in particular aromatic amines) have a considerable sensitization potential, as a result of which skin irritations may arise in people with a corresponding disposition.

An object of the present invention is therefore to provide a permanent hair colouring system consisting of oxime compounds and carbonyl compounds with which the colorations achieved can on the one hand be applied gently to the fibres, and which is acceptable from a toxicological and dermatological point of view, and on the other hand also permits an intense and brilliant colour result. Furthermore, the resulting hair coloration must have good light fastness and resistance to shampooing (durability of the coloration) and a good rubbing fastness.

Surprisingly, it has now been found that fashionable and luminous colour shades can be produced on keratin-containing fibres if a reductive colorant is applied to the fibres which comprises certain oxime compounds, reactive carbonyl compounds and ascorbic acid (vitamin C). The oxime compounds are reduced to the corresponding amines, which in turn react straightaway with the reactive carbonyl compounds in the hair to give coloured substances. The resulting colorations are extremely resistant to washing and durable. Although of course no hydrogen peroxide (oxidizing agent!) can be used during the reduction process, lightening of the natural colour shade, if this is desired, is possible but not necessarily required in the course of a pretreatment.

The use of reactive carbonyl compounds in oxidative hair dyes is disclosed in EP-A-873 744, WO-A-00/33799 and WO-A-2004/022016. The use of ascorbic acid as reducing agent in oxidative hair dyes is disclosed in US-A-3 337 411. WO-A-01/13882 discloses the use of oxime compounds for lightening keratinous tissue.

The oxime compounds according to the invention can be produced by nitrosation of CH-acidic compounds. CH-acidic compounds are generally regarded as being those compounds which carry a hydrogen atom bonded to an aliphatic carbon atom where, on account of the electron-withdrawing substituents, activation of the corresponding carbon-hydrogen bond is effected. Reactive carbonyl compounds according to the invention have at least one carbonyl group as reactive group.

The present invention therefore provides an agent for colouring keratin-containing fibres, in particular human hair, which is characterized in that it comprises a) ascorbic acid (vitamin C) as reducing agent, b) at least one oxime compound of the formula **A1-A23** as "developer substance" and c) at least one reactive carbonyl compound of the formula **B1**-**B17** as "coupler substance".

The oxime compounds **A1-A23** according to the invention are defined as follows:

### Compounds with the formula A1:

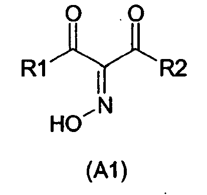

in which **R1** is a cyano group, a substituted or unsubstituted aryl group or a substituted or unsubstituted, saturated or unsaturated heterocycle or a group -C-(O)-R3, where **R3** is a hydrogen atom or a C₁-C₆-alkyl group; and
**R2** is a cyano group, a substituted or unsubstituted C₁-C₆-alkyl group, a C₃-C₆-cydoalkyl group, a substituted or unsubstituted aryl group or a substituted or unsubstituted aminoaryl group or a substituted or ' unsubstituted, saturated or unsaturated heterocycle.

### Compounds with the formula A2:

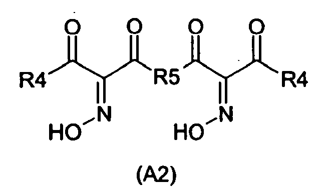

in which **R4** is a cyano group, a substituted or unsubstituted aryl group, a substituted or unsubstituted, saturated or unsaturated heterocycle or a group -C-(O)-R6, where **R6** is a hydrogen atom or a C₁-C₆-alkyl group; and
**R5** is a substituted or unsubstituted C₂-C₆-alkyl group, a C₃-C₆-cycloalkyl group, a substituted or unsubstituted aryl group, a substituted or unsubstituted aminoaryl group, a substituted or unsubstituted, saturated or unsaturated heterocycle.

### Compounds with the formulae A3/A4:

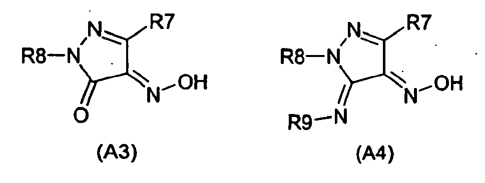

in which **R7** and **R8** may be identical or different and, independently of one another, are a substituted or unsubstituted aryl group, an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cycloalkyl group, a substituted or unsubstituted aminoaryl group, where **R7** can likewise be a C₁-C₆-alkoxy group; and
**R9** is a hydrogen atom or a substituted or unsubstituted C₁-C₆-alkyl group.

### Compounds with the formula A5:

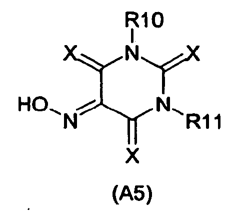

in which **R10** and **R11** may be identical or different and, independently of one another, are a substituted or unsubstituted aryl group, an aryl-C₁-C₄-alkyl group, a C₁-C₆-alkyl group, a C₃-C₆-cycloalkyl group, a C₂-C₆-alkylene group or a bicycle bonded via the radicals R10 and R11; and **X** is an oxygen atom or a sulphur atom.

### Compounds with the formula A6:

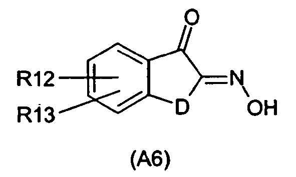

in which **D** is an oxygen atom, a sulphur atom, a sulphoxyl group, a sulphonyl group or a group N-R₁₂ₐ, where **R₁₂ₐ** is a hydrogen atom or a substituted or unsubstituted C₁-C₆-alkyl group;
and **R12** and **R13,** independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR14R15, where **R14** and **R15,** independently of one another, are a hydrogen atom or a C₁-C₆-alkyl group.

### Compounds with the formulae A7/A8:

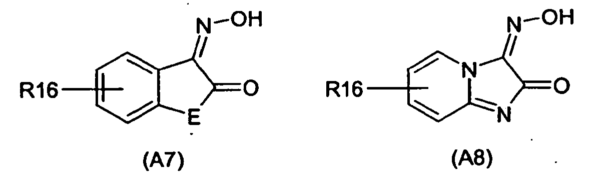

in which **E** is an oxygen atom, a sulphur atom or a group NR17, where **R17** is a hydrogen atom or a substituted or unsubstituted C₁-C₆-alkyl group; and
**R16** is a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR18R19, where **R18** and **R19,** independently of one another, are a hydrogen atom or a C₁-C₆-alkyl group.

### Compounds with the formula A9:

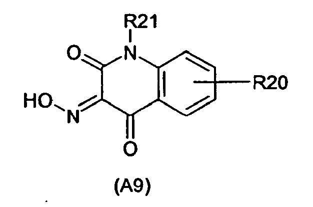

in which **R20** is a hydrogen atom, a halogen atom, a hydroxyl group, a nitro group, a C₁-C₆-alkoxy group or a substituted or unsubstituted C₁-C₆-alkyl group; and
**R21** is a hydrogen atom or a C₁-C₄-alkyl group.

### Compounds of the formulae A10, A11, A12:

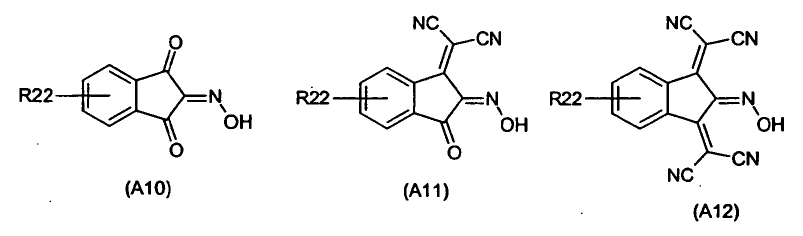

in which **R22** is a hydrogen atom, a halogen atom, a hydroxyl group, a nitro group, a C₁-C₆-alkyl group, a C₁-C₆-alkoxy group, a carboxyl group, a carboxamide group, a cyano group or an amino group -NR23R24, where **R23** and **R24,** independently of one another, are a hydrogen atom or a C₁-C₆-alkyl group.

### Compounds with the formula A13:

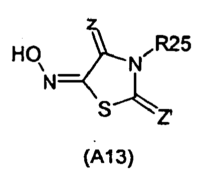

in which **Z** is an oxygen atom or a group -NR26, where **R26** is a hydrogen atom or a C₁-C₆-alkyl group;
**Z'** is a sulphur atom or a group -NR27, where **R27** is a hydrogen atom or a C₁-C₆-alkyl group; and
**R25** is a hydrogen atom, a C₁-C₆-alkyl group or a C₁-C₄-carboxyalkyl group.

### Compounds with the formula A14:

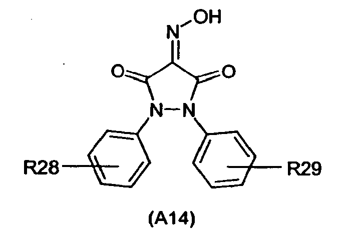

in which **R28** and **R29** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR30R31, where **R30** and **R31,** independently of one another, are a hydrogen atom or a C₁-C₆-alkyl group.

### Compounds with the formula A15:

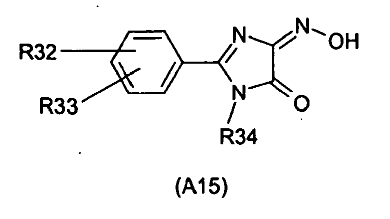

in which **R32** and **R33** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR35R36, where **R35** and **R36,** independently of one another, are a hydrogen atom or a C₁-C₆-alkyl group; and
**R34** is a hydrogen atom or a C₁-C₆-alkyl group.

### Compounds with the formula A16:

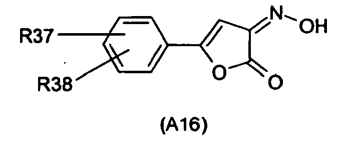

in which **R37** and **R38** may be identical or different and, independently of one another, are a hydrogen atom, a fluorine atom, a chlorine atom, a bromine atom, a hydroxyl group, a nitro group, a C₁-C₆-alkyl group, a-C₁-C₆-alkoxy group, a carboxamide group, a sulphonamide group, a carboxyl group, a C₁-C₄-acyl group, a cyano group or an amino group -NR39R40, where **R39** and **R40,** independently of one another, are a hydrogen atom or a C₁-C₆-alkyl group.

### Compounds with the formulae A17, A18, A19:

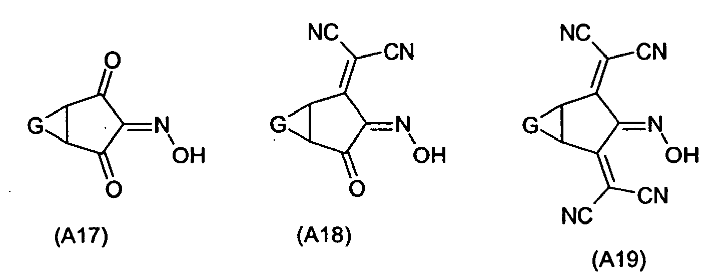

in which **G** is a fused, substituted or unsubstituted aromatic or heteroaromatic ring to which a further aromatic or heteroaromatic ring may additionally be fused.

### Compounds with the formula A20:

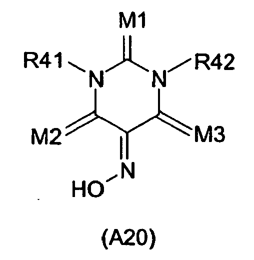

in which **R41** and **R42** may be identical or different and, independently of one another, are a hydrogen atom or a substituted or unsubstituted C₁-C₆-alkyl group;
**M1** is an oxygen atom, a sulphur atom or an NH group;
and **M2** and **M3** may be identical or different and, independently of one another, are an oxygen atom or an NH group.

### 1,3-Dihydro-2H-inden-2-one oxime with the formula A21:

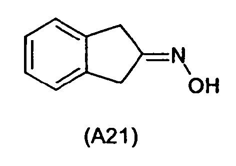

### Compounds with the formulae A22, A23:

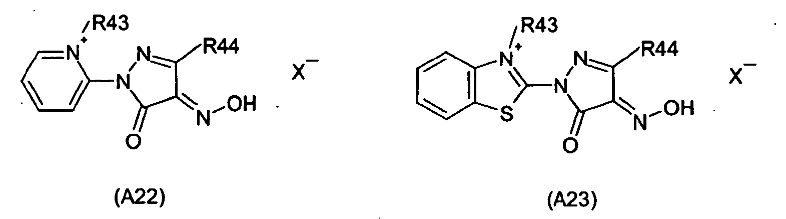

in which **R43** is a substituted or unsubstituted C₁-C₆-alkyl group; and **R44** is a substituted or unsubstituted aryl group or an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cycloalkyl group, a C₁-C₆-alkoxy group or a substituted or unsubstituted aminoaryl group.

The counterions **X⁻** used are preferably sulphate anions, methylsulphate anions, phosphate anions, hydrogenphosphate anions, oxalate anions, formate anions, acetate anions, citrate anions, tartrate anions, malonate anions, pyruvate anions or halogen anions, particular preference being given to the chloride anion, bromide anion and methylsulphate anion.

The oxime compounds (developer substances) of the general formula **A1-A23** are preferably chosen from diethyl 2-(hydroxyimino)-malonate, 1*H*-indene-1,2,3-trione 2-oxime, (2*Z*)-5-(dimethylamino)-1*H-*indene-1,2,3-trione 2-oxime, (2*Z*)-4,5-dimethoxy-1*H*-indene-1,2,3-trione 2-oxime, (2*Z*)-5-methoxy-1*H*-indene-1,2,3-trione 2-oxime, (2*Z*)-5-nitro-1*H*-indene-1,2,3-trione 2-oxime, (2*Z*)-4,6-dichloro-1*H*-indene-1,2,3-trione 2-oxime, (6*Z*)-5H-cyclopenta[*c*]pyridine-5,6,7-trione 6-oxime, (6*Z*)-5*H*-cyclopenta[*b*]pyridine-5,6,7-trione 6-oxime, (2*E*)-1*H*-cyclopenta[*a*]naphthalene-1,2,3-trione 2-oxime, 1*H*-cyclopenta[*b*]quinoxaline-1,2,3-trione 2-oxime, (2*Z*)-4-methyl-4,8b-dihydrocyclopenta[*b*]indole-1,2,3(3a*H*)-trione 2-oxime, (5*E*)-4*H*-cyclopenta[*b*]thiophene-4,5,6-trione 5-oxime, (2*Z*)-5-methyl-1*H*-indene-1,2,3-trione 2-oxime, (2*E*)4,6-dichloro-1*H*-indene-1,2,3-trione 2-oxime, 4,7-dichloro-1*H*-indene-1,2,3-trione 2-oxime, (2*Z*)-1*H*-cyclopenta-[*b*]quinoline-1,2,3-trione 2-oxime, (2*Z*)-1-benzothiophene-2,3-dione 2-oxime 1,1-dioxide, (2*Z*)-1-benzothiophene-2,3-dione 2-oxime, (2*Z*)-1-benzofuran-2,3-dione 2-oxime, (3*E*)-imidazo[1,2-*a*]pyridine-2,3-dione 3-oxime, (3*Z*)-1*H*-indole-2,3-dione 3-oxime, (3*Z*)-1-methyl-1*H*-indole-2,3-dione 3-oxime, (3*Z*)-6-chloro-1-methyl-1*H*-indole-2,3-dione 3-oxime, (3*E*)-7-chloroimidazo[1,2-*a*]pyridine-2,3-dione 3-oxime, 1,2-diphenyl-3,4,5-pyrazolidinetrione-4-oxime, 1*H*-cyclopenta[*b*]naphthalene-1,2,3-trione 2-oxime, 2-[(2*E*)-2-(hydroxyimino)-3-oxo-2,3-dihydro-1*H-*indene-1-ylidene]malononitrile, (4*Z*)-3-methyl-1-phenyl-1*H*-pyrazole-4,5-dione 4-oxime, 1 ,3-diethyl-2-thioxodihydro-4,5,6(1*H*-pyrimidinetrione 5-oxime, (5*Z*)-2-thioxo-1,3-thiazolidine-4,5-dione 5-oxime, (4*Z*)-3-methyl-1-(4-nitrophenyl)-1*H*-pyrazole-4,5-dione 4-oxime, 1,3-dihydro-2*H*-inden-2-one oxime, 4,5,6,7-tetrachloro-1*H*-indene-1,2,3-trione 2-oxime, 2-[(4*E*)-4-(hydroxyimino)-3-methyl-5-oxo-4,5-dihydro-1*H*-pyrazol-1-yl]-1-methylpyridinium methylsulphate and 2-[(4*E*)-4-(hydroxyimino)-3-methyl-5-oxo-4,5-dihydro-1*H*-pyrazol-1-yl]-3-methyl-1,3-benzothiazol-3-ium methylsulphate.

The carbonyl compounds **B1-B16** according to the invention are defined as follows:

### Compounds with the formula B1:

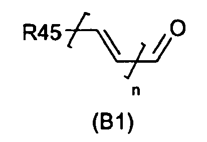

in which **R45** is a substituted or unsubstituted aryl group to which a further aromatic ring may optionally also be fused, or a substituted or unsubstituted, saturated or unsaturated heterocycle; and n is 0, 1, 2 or 3.

### Compounds with the formula B2:

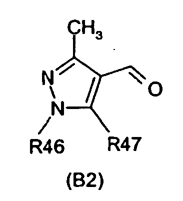

in which **R46** is a C₁-C₆-alkyl group, a substituted or unsubstituted aryl radical or a substituted or unsubstituted, saturated or unsaturated heterocycle; and
**R47** is a pyrrole radical, an imidazole radical, a pyrazole radical, an indole radical, a pyrrolidine radical, a morpholine radical, a dimethylamine radical, a phenol radical or a thiophenol radical, where these radicals are in each case bonded to the general formula **B2** via the heteroatom.

### Pyridoxal (vitamin B₆) with the formula B3:

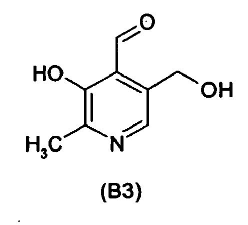

### All-trans-retinal (vitamin A) with the formula B4:

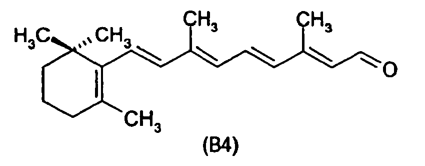

### Compounds with the formula B5:

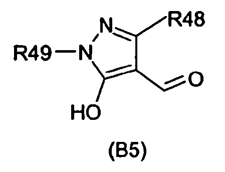

in which **R48** and **R49** may be identical or different and, independently of one another, are a substituted or unsubstituted aryl group, an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cydoalkyl group or a substituted or unsubstituted aminoaryl group, and **R48** can likewise be a C₁-C₆-alkoxy group.

### Compound with the formula B6:

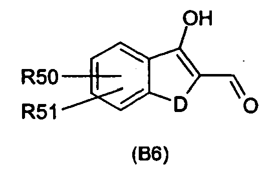

in which **D** has the abovementioned meaning; and
**R50** and **R51** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR52R53, where **R52** and **R53,** independently of one another, are a hydrogen atom or a C₁-C₆-alkyl group.

### Compounds with the formulae B7/B8:

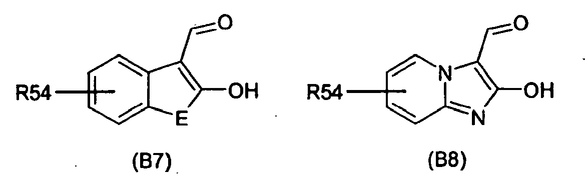

in which **E** has the abovementioned meaning; and
**R54** is a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR55R56, where **R55** and **R56**, independently of one another, are a hydrogen atom or a C₁-C₆-alkyl group.

### Compounds with the formula B9:

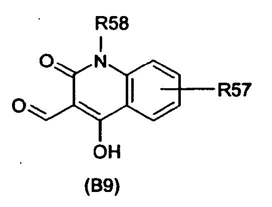

in which **R57** is a hydrogen atom, a halogen atom, a hydroxyl group, a nitro group, a C₁-C₆-alkoxy group or a substituted or unsubstituted C₁-C₆-alkyl group; and
**R58** is a hydrogen atom or a C₁-C₄-alkyl group.

### Compounds with the formulae B10/B11:

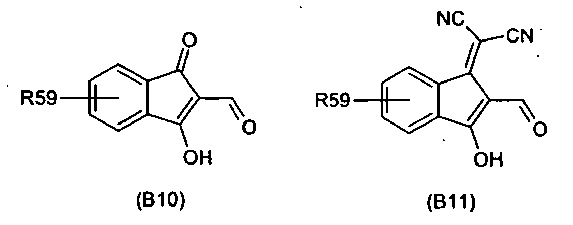

in which **R59** is a hydrogen atom, a halogen atom, a hydroxyl group, a nitro group, a C₁-C₆-alkyl group, a C₁-C₆-alkoxy group, a carboxyl group, a carboxamide group, a cyano group or an amino group -NR60R61, where **R60** and **R61,** independently of one another, are a hydrogen atom or a C₁-C₆-alkyl group.

### Compounds with the formula B12:

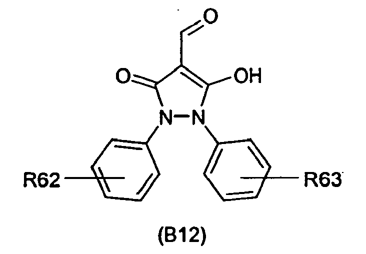

in which **R62** and **R63** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR64R65, where **R64** and **R65**, independently of one another, are a hydrogen atom or a C₁-C₆-alkyl group.

### Compounds with the formulae B13/B14 :

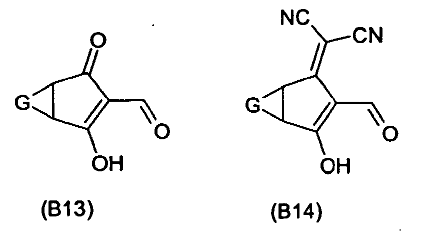

in which **G** has the abovementioned meaning.

### Compounds with the formulae B15/B16:

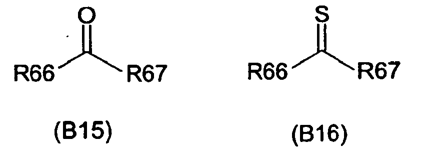

in which **R66** and **R67** may be identical or different and, independently of one another, are a C₁-C₆-alkyl group or a substituted or unsubstituted aryl group.

### Compounds with the formula B17:

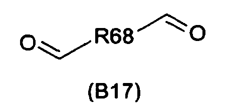

in which **R68** is a substituted or unsubstituted C₁-C₆-alkyl group, a C₁-C₆-alkylene group or a substituted or unsubstituted aryl group.

The reactive carbonyl compounds (coupler substances) of the general formulae **B1-B17** are preferably chosen from benzaldehyde, 4-methoxybenzaldehyde, 4-hydroxybenzaldehyde, 3,4-dimethoxybenzaldehyde, 2,5-dimethoxybenzaldehyde, 3-hydroxy-4-methoxybenzaldehyde, 4-nitrobenzaldehyde, 4-hydroxy-3-methoxybenzaldehyde, 4-(dimethylamino)benzaldehyde, (2*E*)-3-[4-(dimethylamino)phenyl]-2-propenal, 2,4,6-trihydroxybenzaldehyde, 3-hydroxy-5-(hydroxymethyl)-2-methylisonicotine aldehyde, all-trans-retinal, 3,5-dioxo-1,2-diphenyl-4-pyrazolidinecarbaldehyde, (2*E*,4*E*)-5-phenyl-2,4-pentadienal, pentanedial, 4-(methylsulphanyl)benzaldehyde, 1-naphthaldehyde, (2*E*)-3-(4-nitrophenyl)-2-propenal, 3-phenoxybenzaldehyde, terephthalaldehyde, 3-hydroxy-1-oxo-1*H*-cyclopenta[*a*]-naphthalene-2-carbaldehyde, 3-hydroxy-1-oxo-1*H*-cyclopenta[*b*]-quinoxaline-2-carbaldehyde, 3-hydroxy-4-methyl-1-oxo-1,3a,4,8b-tetra-hydrocyclopenta[*b*]indole-2-carbaldehyde, 4-hydroxy-6-oxo-6H-cyclopenta[*b*]thiophene-5-carbaldehyde, 3-hydroxy-5-nitro-1-oxo-1*H*-indene-2-carbaldehyde, 4,6-dichloro-3-hydroxy-1-oxo-1*H*-indene-2-carbaldehyde, 7-hydroxy-5-oxo-5*H*-cyclopenta[*c*]pyridine-6-carbaldehyde, 7-hydroxy-5-oxo-5*H*-cyclopenta[*b*]pyridine-6-carbaldehyde, 3-hydroxy-1-oxo-1*H*-indene-2-carbaldehyde, 5-(dimethylamino)-3-hydroxy-1-oxo-1*H-*indene-2-carbaldehyde, 3-hydroxy-4,5-dimethoxy-1-oxo-1*H*-indene-2-carbaldehyde, 3-hydroxy-5-methoxy-1-oxo-1*H*-indene-2-carbaldehyde, 3-methyl-5-(4-morpholinyl)-1-phenyl-1*H*-pyrazole-4-carbaldehyde, 3-methyl-1-phenyl-5-(1*H*-pyrro)-1-yl)-1*H*-pyrazole-4-carbaldehyde, 3-methyl-1-phenyl-5-(1-pyrrolidinyl)-1*H*-pyrazol-4-carbaldehyde, 3-methyl-1-(2-pyridinyl)-5-(1-pyrrolidinyl)-1*H*-pyrazole-4-carbaldehyde, 3-methyl-1-(2-pyridinyl)-5-(1*H*-pyrrol-1-yl)-1*H*-pyrazole-4-carbaldehyde, 4-methoxy-1-naphthaldehyde, 1*H*-indole-3-carbaldehyde, bis[4-(dimethylamino)-phenyl]methanethione, 2,4-dihydroxybenzaldehyde, 1-methyl-1*H*-indole-3-carbaldehyde and 3,4-dihydroxybenzaldehyde.

The oxime compounds according to the invention of the general formula **A1-A23** can be prepared by nitrosation of the CH-acidic starting compounds. These are either available commercially or are accessible through standard operations from components which are commercially available or can be prepared easily. Thus, for example, the corresponding oxime compound can be prepared from 1,3-indanedione with sodium nitrite in hydrochloric solution (scheme 1).

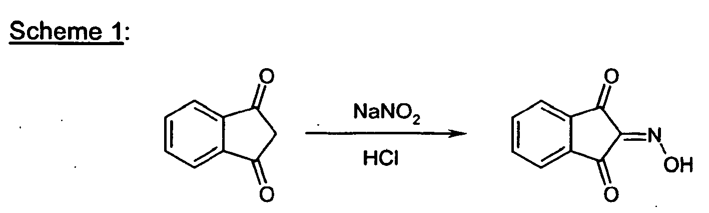

Substituted 1,3-indanediones can be prepared, for example, in accordance with information in the literature (G. Sartori et al., J. Chem. Soc. Perkin. Trans. 1, 1992, 2985-2988) by Friedel-Crafts cycloacylation (cross condensation) from aromatic carbonyl chlorides, anhydrous aluminium chloride and malonyl dichloride (scheme 2).

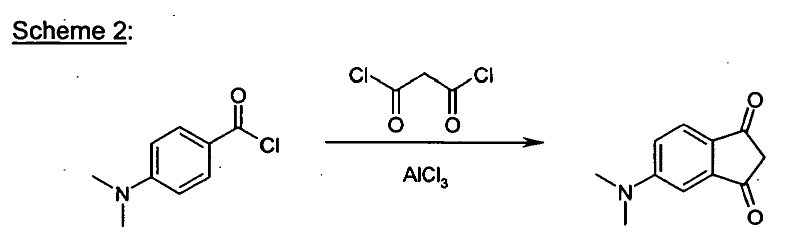

The oxime compounds resulting therefrom can be prepared by nitrosation of the substituted indanedione derivatives with neopentyl nitrite in solvents such as, for example, acetonitrile.

Aromatic and heteroaromatic aldehydes, bisaldehydes and ketones (reactive carbonyl compounds of the general formulae **B1-B17**) are commercially available in large numbers or obtainable from standard reactions. Carbaldehydes are likewise accessible by standard operations from the corresponding CH-acidic compounds.

1,3-Indanedione-2-carbaldehyde can be readily prepared, for example, in accordance with Gudrinietse et al. (Zhurnal Organicheskoi Khimii, Vol. 9, No. 2, 1973, 336-338) by an aldol condensation of 1,3-indanedione with chloral and subsequent alkaline hydrolysis (scheme 3).

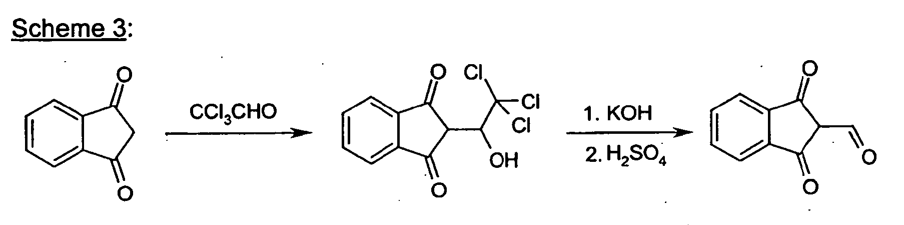

A large number of carbaldehydes can be prepared analogously to this reaction from various CH-acidic compounds.

During the colouring process, the oxime compounds according to the invention of the general formula **A1-A23** are reduced to give the corresponding amines, which, in the hair, react very rapidly with the carbonyl compounds according to the invention of the general formula **B1**-**B17** to give the corresponding coloured compounds (azomethines or Schiffs bases) (scheme 4).

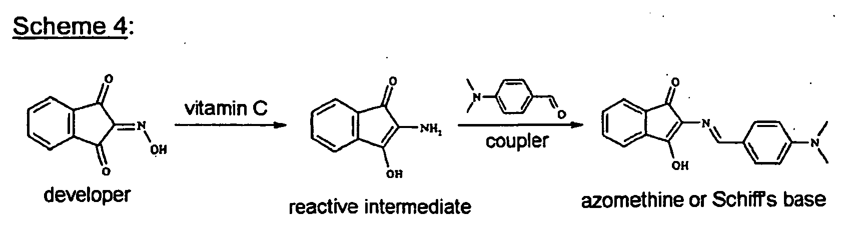

The oxime compounds according to the invention of the general formula **A1-A23** and the carbonyl compounds according to the invention of the general formula **B1-B17** are present in the colorants according to the invention preferably in a total amount of from 0.01 to 10 per cent by weight, in particular 0.1 to 8 per cent by weight.

To produce special colour nuances, besides the components used according to the invention, it is possible to additionally also add to the agents according to the invention one or more customary direct dyes from the group of acidic and basic dyes, nitro dyes, azo dyes, anthraquinone dyes and triphenylmethane dyes, such as for example, 1,4-bis[(2-hydroxyethyl)amino]-2-nitrobenzene, 1-(2-hydroxyethyl)amino-2-nitro-4-[di(2-hydroxyethyl)amino]benzene, (HC Blue No. 2), 1-amino-3-methyl-4-[(2-hydroxyethyl)amino]-6-nitrobenzene, (HC Violet No. 1), 4-[ethyl-(2-hydroxyethyl)amino]-1-[(2-hydroxyethyl)amino]-2-nitrobenzene hydrochloride (HC Blue No. 12), 1-[(2,3-dihydroxypropyl)amino]-4-[methyl-(2-hydroxyethyl)amino]-2-nitrobenzene (HC Blue No. 10), 1-[(2,3-dihydroxypropyl)amino]-4-[ethyl-(2-hydroxyethyl)amino]-2-nitrobenzene hydrochloride (HC Blue No. 9), 1-(3-hydroxypropylamino)-4-[di-(2-hydroxyethyl)amino]-2-nitrobenzene, (HC Violet No. 2); 1-amino-4-[(2-hydroxyethyl)amino]-2-nitrobenzene (HC Red No. 7), 2-amino-4,6-dinitrophenol, 1,4-diamino-2-nitrobenzene (Cl76070), 4-amino-2-nitrodiphenylamine (HC Red No. 1), 1-amino-4-[di(2-hydroxyethyl)amino]-2-nitrobenzene hydrochloride (HC Red No. 13), 1-amino-5-chloro-4-[(2-hydroxyethyl)amino]-2-nitrobenzene, 4-amino-1-[(2-hydroxyethyl)amino]-2-nitrobenzene (HC Red No. 3), 4-amino-2-nitro-1-((prop-2-en-1-yl)amino)benzene, 4-amino-3-nitrophenol, 4-[(2-hydroxyethyl)amino]-3-nitrophenol, 4-[(2-nitrophenyl)amino]phenol (HC Orange No. 1), 1-[(2-aminoethyl)amino]-4-(2-hydroxyethoxy)-2-nitrobenzene (HC Orange No. 2), 4-(2,3-dihydroxypropoxy)-1-[(2-hydroxyethyl)amino]-2-nitrobenzene, (HC Orange No. 3), 1-amino-5-chloro-4-[(2,3-dihydroxypropyl)amino]-2-nitrobenzene (HC Red No. 10), 5-chloro-1,4-[di(2,3-dihydroxypropyl)amino]-2-nitrobenzene (HC Red No. 11), 2-[(2-hydroxyethyl)amino]-4,6-dinitrophenol, 4-ethylamino-3-nitrobenzoic acid, 2-[(4-amino-2-nitrophenyl)amino]benzoic acid, 2-chloro-6-ethylamino-4-nitrophenol, 2-amino-6-chloro-4-nitrophenol, 4-[(3-hydroxypropyl)amino]-3-nitrophenol, 2,5-diamino-6-nitropyridine, 3-amino-6-(methylamino)-2-nitropyridine, 1,2,3,4-tetrahydro-6-nitroquinoxaline, 7-amino-3,4-dihydro-6-nitro-2H-1,4-benzoxazine (HC Red No. 14), 1,2-diamino-4-nitrobenzene (C176020), 1-amino-2-[(2-hydroxyethyl)amino]-5-nitrobenzene (HC Yellow No. 5), 1-(2-hydroxyethoxy)-2-[(2-hydroxyethyl)amino]-5-nitrobenzene, (HC Yellow No. 4), 1-[(2-hydroxyethyl)amino]-2-nitrobenzene (HC Yellow No. 2), 2-[(2-hydroxyethyl)amino]-1-methoxy-5-nitrobenzene, 2-amino-3-nitrophenol, 1-amino-2-methyl-6-nitrobenzene, 1-(2-hydroxyethoxy)-3-methylamino-4-nitrobenzene, 2,3-(dihydroxypropoxy)-3-methylamino-4-nitrobenzene, 2-[(2-hydroxyethyl)amino]-5-nitrophenol (HC Yellow No. 11), 3-[(2-aminoethyl)amino]-1-methoxy-4-nitrobenzene hydrochloride, (HC Yellow No. 9), 1-[(2-ureidoethyl)amino]-4-nitrobenzene, 4-[(2,3-dihydroxypropyl)amino]-3-nitro-1-trifluoromethylbenzene, (HC Yellow No. 6), 1-chloro-2,4-bis[(2-hydroxyethyl)amino]-5-nitrobenzene (HC Yellow No. 10), 4-[(2-hydroxyethyl)amino]-3-nitro-1-methylbenzene, 1-chloro-4.-[(2-hydroxyethyl)amino]-3-nitrobenzene (HC Yellow No. 12), 4-[(2-hydroxyethyl)amino]-3-nitro-1-trifluoromethylbenzene, (HC Yellow No. 13), 4-[(2-hydroxyethyl)amino]-3-nitrobenzonitrile (HC Yellow No. 14), 4-[(2-hydroxyethyl)amino]-3-nitrobenzamide (HC Yellow No. 15), 2,4-dinitro-1-hydroxynaphthalene, 1,4-di[(2,3-dihydroxypropyl)amino]-9,10-anthraquinone, 1,4-di[(2-hydroxyethyl)amino]-9,10-anthraquinone (C161545, Disperse Blue 23), 1-amino-4-hydroxy-9,10-anthraquinone (C160710, Disperse Red 15), 1-hydroxy-4-[(4-methyl-2-sulphophenyl) amino]-9,10-anthraquinone, 7-beta-D-glucopyranosyl-9,10-dihydro-1-methyl-9,10-dioxo-3,5,6,8-tetrahydroxy-2-anthracenecarboxylic acid (C175470, Natural Red 4), 1-[(3-aminopropyl)amino]-9,10-anthraquinone (HC Red No. 8), 1,4-diamino-9,10-anthraquinone (CI61100, Disperse Violet No. 1), 1-amino-4-(methylamino)-9,10-anthraquinone (C161105, Disperse Violet No. 4, Solvent Violet No. 12), N-(6-((3-chloro-4-(methylamino)phenyl)imino-4-methyl-3-oxo-1,4-cyclohexadien-1-yl)urea (HC Red No. 9), 2-((4-(di(2-hydroxyethyl)amino)phenyl)aminoy5-((2-hydroxyethyl)amino)-2,5-cyclohexadiene-1,4-dione (HC Green No. 1), 2-hydroxy-1,4-naphthoquinone (CI75480, Natural Orange No. 6), 1,2-dihydro-2-(1,3-dihydro-3-oxo-2H-indol-2-ylidene)-3H-indol-3-one (C173000), 1,3-bis(dicyanomethylene)indane, di[4-(diethylamino)phenyl]-[4-(ethylamino)naphthyl]carbenium chloride (C142595; Basic Blue No. 7), di[4-(dimethylamino)phenyl][4-(phenylamino)naphthyl]carbenium chloride (C144045; Basic Blue No. 26), Basic Blue No. 77, 8-amino-2-bromo-5-hydroxy-4-imino-6-[(3-(trimethylammonio)phenyl)amino]-1 (4H)-naphthalinone chloride (CI56059; Basic Blue No. 99), tri(4-amino-3-methylphenyl)carbenium chloride (C142520; Basic Violet No. 2), di(4-aminophenyl)(4-amino-3-methylphenyl)carbenium chloride (CI42510; Basic Violet No. 14), 1-[(4-aminophenyl)azo]-7-(trimethylammonio)-2-naphthol chloride (C112250; Basic Brown No. 16), 3-[(4-amino-2,5-dimethoxyphenyl)azo]-N,N,N-trimethylbenzenaminium chloride (CI112605, Basic Orange No. 69), 1-[(4-amino-2-nitrophenyl)azo]-7-(trimethylammonio)-2-naphthol chloride (Basic Brown No. 17), 1-[(4-amino-3-nitrophenyl)azo]-7-(trimethylammonio)-2-naphthol chloride (CI12251; Basic Brown No. 17), 2-((4-aminophenyl)azo)-1,3-dimethyl-1H-imidazol-3-ium chloride (Basic Orange No. 31), 3,7-diamino-2,8-dimethyl-5-phenylphenazinium chloride (C150240; Basic Red No.2), 1,4-dimethyl-5-[(4-(dimethylamino)phenyl)azo]-1 ,2,4-triazolium chloride (CI11055; Basic Red No. 22), 1 ,3-dimethyl-2-((4-dimethylamino)phenyl)azo-1H-imidazol-3-ium chloride (Basic Red No. 51), 2-hydroxy-1-[(2-methoxyphenyl)azo]-7-(trimethylammonio)naphthalene chloride (Cl12245; Basic Red No. 76), 3-methyl-1-phenyl-4-[(3-(trimethylammonio)phenyl)azo]-pyrazol-5-one chloride (Cl12719; Basic Yellow No. 57), 1-methyl-4-((methylphenylhydrazono)methyl)pyridinium methylsulphate (Basic Yellow No. 87), 1-(2-morpholiniumpropylamino)-4-hydroxy-9,10-anthraquinone methylsulphate, 1-[(3-(dimethylpropylaminium)propyl)amino]-4-(methylamino)-9,10-anthraquinone chloride, 1-[di(2-hydroxyethyl)amino]-3-methyl-4-[(4-nitrophenyl)azo]benzene (Cl11210, Disperse Red No. 17), 1-[di(2-hydroxyethyl)amino]-4-[(4-nitrophenyl)azo]benzene, (Disperse Black No. 9), 4-[(4-aminophenyl)azo]-1-[di(2-hydroxyethyl)amino]-3-methylbenzene, (HC Yellow No. 7), 2,6-diamino-3-[(pyridin-3-yl)azo]-pyridine and 2-((4-(ethyl(2-hydroxyethyl)amino)-2-methylphenyl)azo)-5-nitro-1,3-thiazole (Cl111935; Disperse Blue No. 106), 3-(2',6'-diaminopyridyl-3'-azo)pyridine (= 2,6-diamino-3-((pyridin-3-yl)azo)pyridine, N,N-di(2-hydroxyethyl)-3-methyl-4-((4-nitrophenyl)azo)aniline (Disperse Red 17, Cl11210), 3-diethylamino-7-(4-dimethylaminophenylazo)-5-phenylphenazinium chloride (Cl 11050), 4-(2-thiazolylazo)resorcinol, 4-((4-phenylamino)azo)benzosulphonic acid sodium salt (Orange IV), 1-((3-aminopropyl)amino)-9,10-anthracenedione, (HC Red No. 8), 3',3",4,5,5',5",6,7-octabromophenol sulphonephthalein (Tetrabromophenol Blue), 1-((4-amino-3,5-dimethylphenyl)-(2,6-dichlorophenyl)methylene)-3,5-dimethyl-4-imino-2,5-cyclohexadiene phosphoric acid (1:1) (Basic Blue 77), 3',3",5',5"-tetrabromo-m-cresol sulphonephthalein, 2,4-dinitro-1-naphthol-7-sulphonic acid disodium salt (Acid Yellow 1, Cl 10316), 4-[2'-hydroxy-1'-naphthyl)azo]benzosulphonic acid sodium salt (Acid Orange 7,Cl 15510), 3',6'-dihydroxy-2',4',5',7'-tetraiodospiro-[isobenzofuran-1 (3H), 9'(9H)-xanthen]-3-one disodium salt (Acid Red 51, Cl 45430), 6-hydroxy-5-((2-methoxy-5-methyl-4-sulphophenyl)azo)-2-naphthalenesulphonic acid disodium salt (FD&C Red 40, Cl 16035), 2,4-dinitro-1-naphthol sodium salt (Acid Yellow 24; Cl 10315), 2',4',5',7'-tetrabromo-4,5,6,7-tetrachloro-3',6'-dihydroxyspiro-(isobenzofuran-1 (3H), 9'[9H]xanthen]-3-one disodium salt (Acid Red 92; Cl 45410), 4-(2-hydroxy-1-naphthylazo)-3-methyl-benzenesulphonic acid sodium salt (Acid Orange 8, Cl 15575), 2-amino-1,4-naphthalenedione, dithizone (1,5-diphenylthiocarbazone), N-(2-hydroxyethyl))-2-nitro-4-trifluoromethyl)aniline (HC Yellow 13), N-(2-hydroxyethyl)-4-nitroaniline and 4-chloro-N-(2,3-dihydroxypropyl)-2-nitroaniline.

The abovementioned direct dyes may be present in a total amount of from 0.01 to 4 per cent by weight, where the total content of dyes in the colorant according to the invention is preferably 0.01 to 10 per cent by weight, in particular 0.1 to 5 per cent weight.

In addition, the colorants according to the invention can also comprise naturally occurring dyes, such as, for example, henna red, henna neutral, henna black, camomile, sandalwood, black tea, buckthorn bark, sage, logwood, madder root, catechu, sedre and alkanna root.

The colorants according to the invention produce intense colorations even at physiologically compatible temperatures of less than 45°C. They are therefore particularly suitable for colouring human hair. For use on human hair, the colorants are usually incorporated into a hydrous cosmetic carrier. Suitable cosmetic carriers are, for example, creams, emulsions, gels or else surfactant-containing foaming solutions, such as, for example, shampoos or other preparations which are suitable for application to keratin-containing fibres. If necessary, it is also possible to incorporate the colorants into anhydrous carriers, powders, pellets or granules.

The colorant according to the invention can further comprise all additives which are customary and known for such preparations, for example perfume oils, complexing agents, waxes, preservatives, thickeners, antioxidants, alginates, guar gum, haircare substances, such as, for example, cationic polymers or lanolin derivatives, or anionic, nonionic, zwitterionic, amphoteric or cationic surface-active substances (surfactants). Preferably, amphoteric or nonionic surface-active substances are used, for example betaine surfactants, propionates and glycinates, such as, for example, cocoamphoglycinates or cocoamphodiglycinates, ethoxylated surfactants with 1 to 1000 ethylene oxide units, preferably with 1 to 300 ethylene oxide units, such as, for example, glyceride alkoxylates, for example castor oil ethoxylated with 25 ethylene oxide units, polyglycolamides, ethoxylated alcohols and ethoxylated fatty alcohols (fatty alcohol alkoxylates) and ethoxylated fatty acid sugar esters, in particular ethoxylated sorbitan fatty acid esters. The abovementioned constituents are used in the amounts customary for such purposes, for example the surface-active substances in a concentration of from 0.1 to 30 per cent by weight, and the care substances in an amount of from 0.1 to 5 per cent by weight.

The colorant according to the invention can, particularly if it is a hair colorant, be in the form of a powder or of granules, which is/are dissolved prior to application in an aqueous or aqueous-alcoholic preparation, or in the form of an aqueous or aqueous-alcoholic solution, a cream, a gel, an emulsion or an aerosol foam, where the hair colorant can be formulated either in the form of a single-component preparation or else in the form of a multicomponent preparation, for example in the form of a two-component preparation, in which case the particular oxime derivatives of the general formula **A1-A23** and the corresponding carbonyl compounds of the general formulae **B1-B17** are packaged separately from the other constituents (e.g. the ascorbic acid) and the ready-to-use hair colorant is only prepared immediately prior to application by mixing the two components.

The colorant according to the invention generally has a pH of from 2 to 6, preferably 3 to 5. Both organic and also inorganic acids are suitable for adjusting the pH according to the invention. Examples of suitable acids are the following acids: α-hydroxycarboxylic acids, such as, for example, glycolic acid, lactic acid, tartaric acid, citric acid or malic acid, gluconolactone, acetic acid, hydrochloric acid or phosphoric acid, and mixtures of these acids. Particular preference here is given to the use of the ascorbic acid used as reducing agent.

The colorant according to the invention is generally used by applying to the hair an amount of the hair colorant adequate for the hair colouring, 30 to 120 grams depending on the length of hair, leaving the hair colorant to act at 15 to 45 degrees Celsius for 1 to 60 minutes, preferably 5 to 30 minutes, then thoroughly rinsing the hair with water, optionally washing with a shampoo and/or after-treating with a hair-conditioning composition and finally drying.

The colorant described above can also comprise natural or synthetic polymers or modified polymers of natural origin customary for cosmetic compositions, through which setting of the hair is achieved at the same time as the colouring. Such compositions are generally referred to as tinting setting compositions or colour setting compositions.

Of the synthetic polymers known for this purpose in cosmetics, mention may be made, for example, of polyvinylpyrrolidone, polyvinyl acetate, polyvinyl alcohol or polyacrylic compounds, such as polyacrylic acid or polymethacrylic acid, basic polymers of esters of polyacrylic acid, polymethacrylic acid and aminoalcohols, for example their salts or quaternization products, polyacrylonitrile, polyvinyl acetates and copolymers of such compounds, such as, for example, polyvinylpyrrolidone-vinyl acetate; whereas natural polymers or modified natural polymers which may be used are, for example, chitosan (deacetylated chitin) or chitosan derivatives. The abovementioned polymers may be present in the colorant according to the invention in the amounts customary for such compositions, in particular in an amount of from about 1 to 5 per cent by weight.

The hair colorant with additional setting is used in a known and customary manner by wetting the hair with the setting composition, fixing (arranging) the hair in the hairstyle and then drying.

The colorant according to the invention permits an even, intense and long-lasting coloration of keratin fibres (for example human hair, wool or furs) without noteworthy discoloration of the skin and/or scalp.

The examples below are intended to illustrate the subject-matter of the invention in more detail without limiting it thereto.

### Examples

### Example 1: Synthesis of 2-[(2E)-2-(hydroxyimino)-3-oxo-2,3-dihydro-1H-inden-1-ylidene]malononitrile (b)

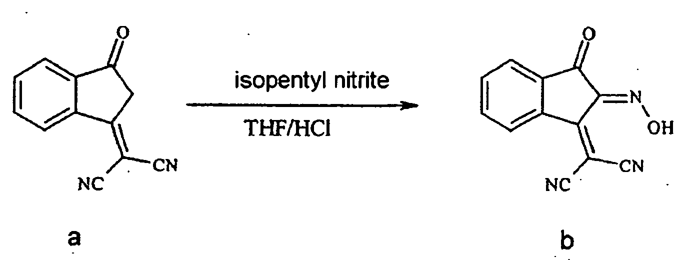

0.20 g (1.03 mmol) of 2-(3-oxo-2,3-dihydro-1*H*-inden-1-ylidene)-malononitrile **(a)** are dissolved in 10 ml of tetrahydrofuran (THF). This produces a clear green solution which is cooled to 0 °C. 0.35 ml (4.12 mmol) of 37% hydrochloric acid are then added. A colour change from green to orange is observed. 0.14 ml (1.03 mmol) of isopentyl nitrite are now added dropwise to the cooled solution, which is stirred for a further 30 minutes at 0 °C until a clear yellow solution has formed. This is left to stand overnight at room temperature and the resulting precipitate is removed by suction. The product **(b)** is isolated as white powder in a yield of 44%.
¹H NMR (d₆-DMSO/300 MHz): δ = 7.42 (s, 4H, aromatic).

### Examples 2 to 18: Hair colorants

| | |
|---|---|
| 5 mmol | oxime compound of the general formula **A1-A23** |
| 5 mmol | carbonyl compounds of the general formula **B1-B17** |
| 5.0 g | ethanol |
| 4.0 g | decylpolyglucose |
| 0.2 g | ethylenediaminotetraacetic acid disodium salt hydrate |
| ad 100.0 g | water, demineralized |

The hair colouring is carried out by applying an amount of the colorant and of the reducing agent ascorbic acid (preferably 1-4 g/10 ml of colouring solution) adequate for the hair colouring to the hair.
After a contact time of 30 minutes at 40 °C, the hair is rinsed with lukewarm water and dried.

The colouring results are summarized in Tables 1, 2 and 3 below.

Unless otherwise indicated, all percentages are by weight.

**Table 1: Oxime compound: 1H-indene-1,2,3-trione 2-oxime**

| **Ex. No.** | **Carbonyl compound** | **Colouring result** |
|---|---|---|
| **2** | 4-Hydroxy-3-methoxybenzaldehyde | golden brown |
| **3** | 4-(Dimethylamino)benzaldehyde | rust red |
| **4** | 4-Methoxy-1-naphthaldehyde | golden brown |
| **5** | (2*E*)-3-[4-Dimethylamino)phenyl]-2-propenal | dark red |
| **6** | 1*H*-indole-3-carbaldehyde | golden yellow |
| **7** | Bis[4-(dimethylamino)phenyl]-methanethione | pale brown |
| **8** | 2,4-Dihydroxybenzaldehyde | golden yellow |
| **9** | 2,4,6-Trihydroxybenzaldehyde | orange yellow |
| **10** | 3-Hydroxy-4-methoxybenzaldehyde | golden brown |

**Table 2: Oxime compound: (4Z)-3-Methyl-1-phenyl-1H-pyrazole-4,5-dione 4-oxime**

| **Ex. No.** | **Carbonyl compound** | **Colouring result** |
|---|---|---|
| **11** | 4-Hydroxy-3-methoxybenzaldehyde | yellow orange |
| **12** | 4-(Dimethylamino)benzaldehyde | orange |
| **13** | 1-Methyl-1*H*-indole-3-carbaldehyde | yellow orange |
| **14** | (2*E*)-3-[4-Dimethylamino)phenyl]-2-propenal | garnet red |
| **15** | 3,4-Dihydroxybenzaldehyde | golden orange |

**Table 3: Oxime compound: 1,3-Dihydro-2H-inden-2-one oxime**

| **Ex. No.** | **Coupler** | **Colouring result** |
|---|---|---|
| **16** | 4-Hydroxy-3-methoxybenzaldehyde | lemon yellow |
| **17** | 4-(Dimethylamino)benzaldehyde | lemon yellow |
| **18** | (2*E*)-3-[4-Dimethylamino)phenyl]-2-propenal | orange |

## Claims

1. Agent for colouring keratin-containing fibres, **characterized in that** it comprises a) ascorbic acid, b) at least one oxime compound of the formula **A1-A23;** 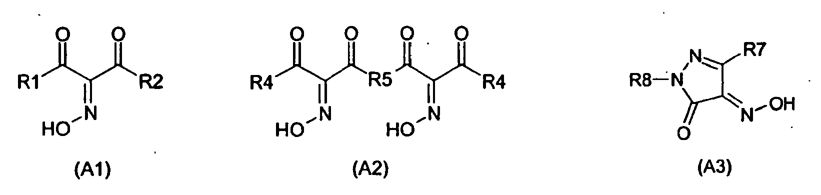 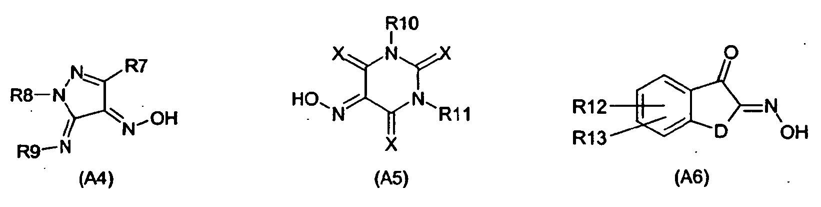 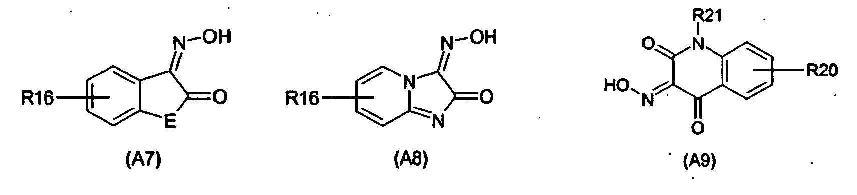 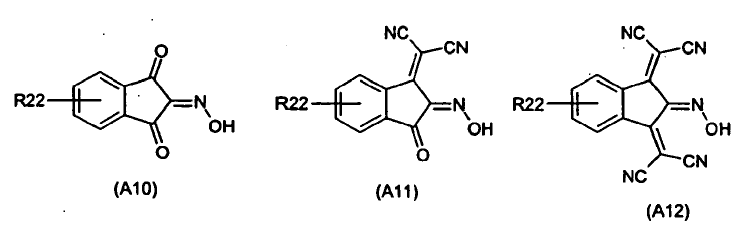 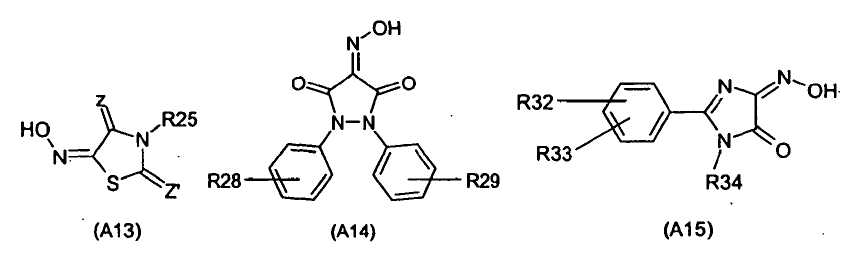 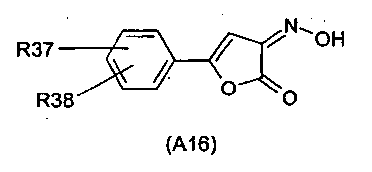 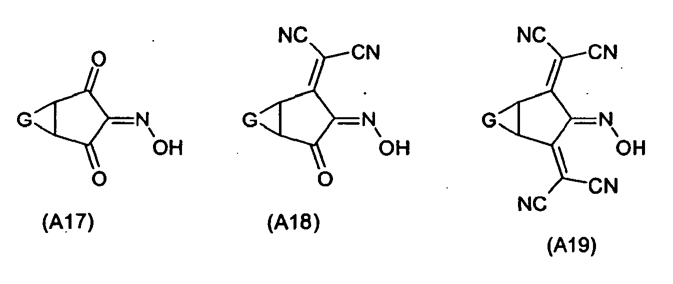 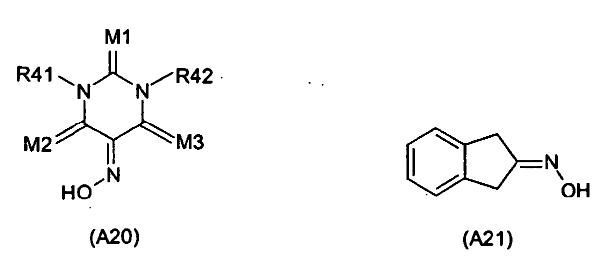 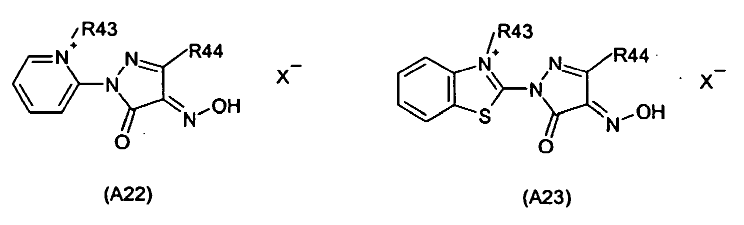 in which
**R1** is a cyano group, a substituted or unsubstituted aryl group or a substituted or unsubstituted, saturated or unsaturated heterocycle or a group -C-(O)-R3, where **R3** is a hydrogen atom or a C₁-C₆-alkyl group;
**R2** is a cyano group, a substituted or unsubstituted C₁-C₆-alkyl group, a C₃-C₆-cycloalkyl group, a substituted or unsubstituted aryl group or a substituted or unsubstituted aminoaryl group or a substituted or unsubstituted, saturated or unsaturated heterocycle;
**R4** is a cyano group, a substituted or unsubstituted aryl group, a substituted or unsubstituted, saturated or unsaturated heterocycle or a group -C-(O)-R6, where **R6** is a hydrogen atom or a C₁-C₆-alkyl group;
**R5** is a substituted or unsubstituted C₂-C₆-alkyl group, a C₃-C₆-cydoalkyl group, a substituted or unsubstituted aryl group, a substituted or unsubstituted aminoaryl group, a substituted or unsubstituted, saturated or unsaturated heterocycle;
in which **R7** and **R8** may be identical or different and, independently of one another, are a substituted or unsubstituted aryl group, an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cycloalkyl group, a substituted or unsubstituted aminoaryl group, where **R7** can likewise be a C₁-C₆-alkoxy group;
**R9** is a hydrogen atom or a substituted or unsubstituted C₁-C₆-alkyl group;
**R10** and **R11** may be identical or different and, independently of one another, are a substituted or unsubstituted aryl group, an aryl-C₁-C₄-alkyl group, a C₁-C₆-alkyl group, a C₃-C₆-cycloalkyl group, a C₂-C₆-alkylene group or a bicycle bonded via the radicals R10 and R11;
**X** is an oxygen atom or a sulphur atom;
**D** is an oxygen atom, a sulphur atom, a sulphoxyl group, a sulphonyl group or a group N-R₁₂ₐ, where **R₁₂ₐ** is a hydrogen atom or a substituted or unsubstituted C₁-C₆-alkyl group;
and **R12** and **R13,** independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR14R15, where **R14** and **R15,** independently of one another, are a hydrogen atom or a C₁-C₆-alkyl group;
**E** is an oxygen atom, a sulphur atom or a group NR17, where **R17** is a hydrogen atom or a substituted or unsubstituted C₁-C₆-alkyl group;
**R16** is a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR18R19, where **R18** and **R19,** independently of one another, are a hydrogen atom or a C₁-C₆-alkyl group;
**R20** is a hydrogen atom, a halogen atom, a hydroxyl group, a nitro group, a C₁-C₆-alkoxy group or a substituted or unsubstiturted C₁-C₆-alkyl group; **R21** is a hydrogen atom or a C₁-C₄-alkyl group;
**R22** is a hydrogen atom, a halogen atom, a hydroxyl group, a nitro group, a C₁-C₆-alkyl group, a C₁-C₆-alkoxy group, a carboxyl group, a carboxamide group, a cyano group or an amino group -NR23R24, where
**R23** and **R24**, independently of one another, are a hydrogen atom or a C₁-C₆-alkyl group;
**Z** is an oxygen atom or a group -NR26, where **R26** is a hydrogen atom or a C₁-C₆-alkyl group;
**Z'** is a sulphur atom or a group -NR27, where **R27** is a hydrogen atom or a C₁-C₆-alkyl group;
**R25** is a hydrogen atom, a C₁-C₆-alkyl group or a C₁-C₄-carboxyalkyl group;
**R28** and **R29** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR30R31, where **R30** and **R31,** independently of one another, are a hydrogen atom or a C₁-C₆-alkyl group;
**R32** and **R33** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR35R36, where
**R35 and R36,** independently of one another, are a hydrogen atom or a C₁-C₆-alkyl group;
**R34** is a hydrogen atom or a C₁-C₆-alkyl group;
**R37** and **R38** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C1-C₄-acyl group, a cyano group or an amino group -NR39R40, where **R39** and **R40,** independently of one another, are a hydrogen atom or a C₁-C₆-alkyl group;
**G** is a fused, substituted or unsubstituted aromatic or heteroaromatic ring to which a further aromatic or heteroaromatic ring may additionally be fused;
**R41** and **R42** may be identical or different and, independently of one another, are a hydrogen atom or a substituted or unsubstituted C₁-C₆-alkyl group;
**M1** is an oxygen atom, a sulphur atom or an NH group;
and **M2** and **M3** may be identical or different and, independently of one another, are an oxygen atom or an NH group;
**R43** is a substituted or unsubstituted C₁-C₆-alkyl group; **R44** is a substituted or unsubstituted aryl group or an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cydoalkyl group, a C₁-C₆-alkoxy group or a substituted or unsubstituted aminoaryl group; and
X⁻ is a counterion;
and c) at least one reactive carbonyl compound of the formula **B1-B17;** 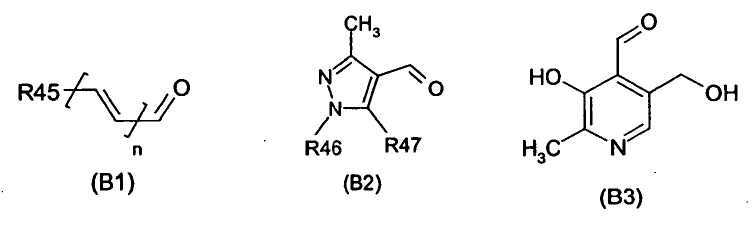 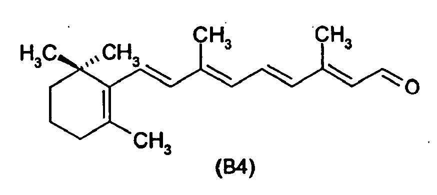 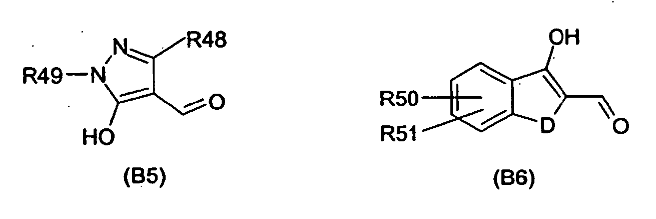 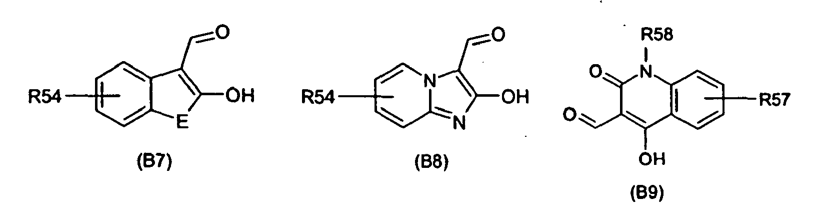 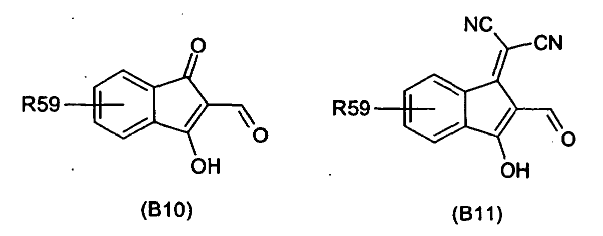 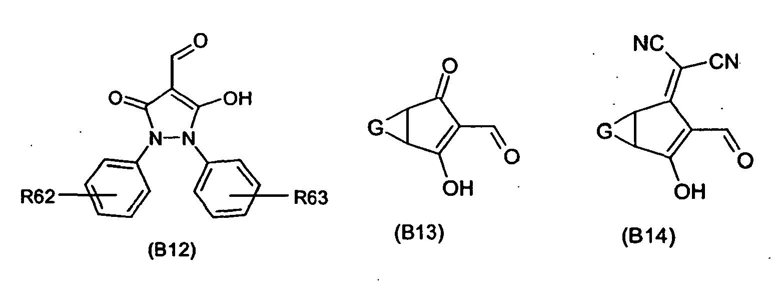 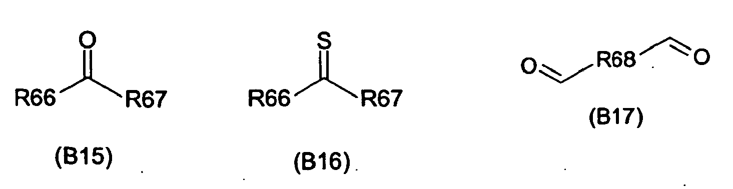 in which
**R45** is a substituted or unsubstituted aryl group to which a further aromatic ring may optionally also be fused, a substituted or unsubstituted, saturated or unsaturated heterocycle;
n is 0,1,2 or 3 ;
**R46** is a C₁-C₆-alkyl group, a substituted or unsubstituted aryl radical or a substituted or unsubstituted, saturated or unsaturated heterocycle;
**R47** is a pyrrole radical, an imidazole radical, a pyrazole radical, a indole radical, a pyrrolidine radical, a morpholine radical, a dimethylamine radical, a phenol radical or a thiophenol radical, where these radicals are in each case bonded to the general formula **B2** via the heteroatom;
**R48** and **R49** may be identical or different and, independently of one another, are a substituted or unsubstituted aryl group, an aryl-C₁-C₄-alkyl group, a substituted or unsubstituted, saturated or unsaturated heterocycle, a substituted or unsubstituted C₁-C₆-alkyl group, an acetyloxy group, a C₃-C₆-cycloalkyl group or a substituted or unsubstituted aminoaryl group, and **R48** can likewise be a C₁-C₆-alkoxy group;
**D** has the abovementioned meaning;
**R50** and **R51** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR52R53, where **R52** and **R53,** independently of one another, are a hydrogen atom or a C₁-C₆-alkyl group;
**E** has the abovementioned meaning;
**R54** is a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR55R56, where **R55** and **R56,** independently of one another, are a hydrogen atom or a C₁-C₆-alkyl group;
**R57** is a hydrogen atom, a halogen atom, a hydroxyl group, a nitro group, a C₁-C₆-alkoxy group or a substituted or unsubstituted C₁-C₆-alkyl group; **R58** is a hydrogen atom or a C₁-C₄-alkyl group;
**R59** is a hydrogen atom, a halogen atom, a hydroxyl group, a nitro group, a C₁-C₆-alkyl group, a C₁-C₆-alkoxy group, a carboxyl group, a carboxamide group, a cyano group or an amino group -NR60R61, where **R60** and **R61,** independently of one another, are a hydrogen atom or a C₁-C₆-alkyl group;
**R62** and **R63** may be identical or different and, independently of one another, are a hydrogen atom, a fluorine atom, a chlorine atom, a bromine atom, a hydroxyl group, a nitro group, a C₁-C₆-alkyl group, a C₁-C₆-alkoxy group, a carboxamide group, a sulphonamide group, a carboxyl group, a C₁-C₄-acyl group, a cyano group or an amino group -NR64R65, where **R64** and **R65,** independently of one another, are a hydrogen atom or a C₁-C₆-alkyl group;
in which **G** has the abovementioned meaning;
**R66** and **R67** may be identical or different and, independently of one another, are a C₁-C₆-alkyl group or a substituted or unsubstituted aryl group; and
**R68** is a substituted or unsubsituted C₁-C₆-alkyl group, a C₁-C₆-alkylene group or a substituted or unsubstituted aryl group.

2. Agent according to Claim 1, **characterized in that** the oxime compound of the general formula **A1-A23** is chosen from diethyl 2-(hydroxyimino)malonate, 1*H*-indene-1,2,3-trione 2-oxime, (2*Z*)-5-(dimethylamino)-1*H*-indene-1,2,3-trione 2-oxime, (2*Z*)-4,5-dimethoxy-1*H-*indene-1,2,3-trione 2-oxime, (2*Z*)-5-methoxy-1*H*-indene-1,2,3-trione 2-oxime, (2*Z*)-5-nitro-1*H*-indene-1,2,3-trione 2-oxime, (2*Z*)-4,6-dichloro-1*H*-indene-1,2,3-trione 2-oxime, (6*Z*)-5*H*-cyclopenta[*c*]pyridine-5,6,7-trione 6-oxime, (6*Z*)-5*H*-cyclopenta[*b*]pyridine-5,6,7-trione 6-oxime, (2*E*)-1*H*-cyclopenta[*a*]-naphthalene-1,2,3-trione 2-oxime, 1*H*-cyclopenta[*b*]quinoxaline-1,2,3-trione 2-oxime, (2*Z*)-4-methyl-4,8b-dihydrocyclopenta[*b*]indole-1,2,3(3a*H*)-trione 2-oxime, (5*E*)-4*H*-cyclopenta[*b*]thiophene-4,5,6-trione 5-oxime, (2*Z*)-5-methyl-1*H*-indene-1,2,3-trione 2-oxime, (2*E*)-4,6-dichloro-1*H-*indene-1,2,3-trione 2-oxime, 4,7-dichloro-1*H*-indene-1,2,3-trione 2-oxime, (2*Z*)-1*H*-cyclopenta[*b*]quinoline-1,2,3-trione 2-oxime, (2*Z*)-1-benzothiophene-2,3-dione 2-oxime 1,1-dioxide, (2*Z*)-1-benzothiophene-2,3-dione 2-oxime, (2*Z*)-1-benzofuran-2,3-dione 2-oxime, (3*E*)-imidazo[1,2-*a*]pyridine-2,3-dione 3-oxime, (3*Z*)-1*H*-indole2,3-dione 3-oxime, (3*Z*)-1-methyl-1*H*-indole-2,3-dione 3-oxime, (3*Z*)-6-chloro-1-methyl-1*H*-indole-2,3-dione 3-oxime, (3*E*)-7-chloroimidazo[1,2-*a*]pyridine-2,3-dione 3-oxime, 1,2-diphenyl-3,4,5-pyrazolidinetrione-4-oxime, 1*H*-cyclopenta[*b*]naphthalene-1,2,3-trione 2-oxime, 2-[(2*E*)-2-(hydroxyimino)-3-oxo-2,3-dihydro-1*H*-indene-1-ylidene]malononitrile, (4Z)-3-methyl-1 -phenyl-1*H*-pyrazole-4,5-dione 4-oxime, 1,3-diethyl-2-thioxodihydro-4,5,6(1*H*)-pyrimidinetrione 5-oxime, (5*Z*)-2-thioxo-1,3-thiazolidine-4,5-dione 5-oxime, (4*Z*)-3-methyl-1-(4-nitrophenyl)-1*H-*pyrazole-4,5-dione 4-oxime, 1,3-dihydro-2*H*-inden-2-one oxime, 4,5,6,7-tetrachloro-1*H*-indene-1,2,3-trione 2-oxime, 2-[(4*E*)-4-(hydroxyimino)-3-methyl-5-oxo-4,5-dihydro-1*H*-pyrazol-1-yl]-1-methyl-pyridinium methylsulphate and 2-[(4*E*)-4-(hydroxyimino)-3-methyl-5-oxo-4,5-dihydro-1*H*-pyrazol-1-yl]-3-methyl-1,3-benzothiazol-3-ium methylsulphate.

3. Agent according to Claim 1, **characterized in that** the reactive carbonyl compound of the general formulae **B1-B17** is chosen from benzaldehyde, 4-methoxybenzaldehyde, 4-hydroxybenzaldehyde, 3,4-dimethoxybenzaldehyde, 2,5-dimethoxybenzaldehyde, 3-hydroxy-4-methoxybenzaldehyde, 4-nitrobenzaldehyde, 4-hydroxy-3-methoxybenzaldehyde, 4-(dimethylamino)benzaldehyde, (2*E*)-3-[4-(dimethylamino)phenyl]-2-propenal, 2,4,6-trihydroxybenzaldehyde, 3-hydroxy-5-(hydroxymethyl)-2-methylisonicotine aldehyde, all-trans-retinal, 3,5-dioxo-1,2-diphenyl-4-pyrazolidinecarbaldehyde, (2*E*,4*E*)-5-phenyl-2,4-pentadienal, pentanedial, 4-(methylsulphanyl)benzaldehyde, 1-naphthaldehyde, (2*E*)-3-(4-nitrophenyl)-2-propenal, 3-phenoxybenzaldehyde, terephthalaldehyde, 3-hydroxy-1-oxo-1*H*-cyclopenta[*a*]-naphthalene-2-carbaldehyde, 3-hydroxy-1-oxo-1*H*-cyclopenta[*b*]-quinoxaline-2-carbaldehyde, 3-hydroxy-4-methyl-1-oxo-1,3a,4,8b-tetrahydrocyclopenta[*b*]indole-2-carbaldehyde, 4-hydroxy-6-oxo-6H-cyclopenta[*b*]thiophene-5-carbaldehyde, 3-hydroxy-5-nitro-1-oxo-1*H*-indene-2-carbaldehyde, 4,6-dichloro-3-hydroxy-1-oxo-1*H*-indene-2-carbaldehyde, 7-hydroxy-5-oxo-5*H*-cyclopenta[*c*]pyridine-6-carbaldehyde, 7-hydroxy-5-oxo-5*H*-cyclopenta[*b*]pyridine-6-carbaldehyde, 3-hydroxy-1-oxo-1*H*-indene-2-carbaldehyde, 5-(dimethylamino)-3-hydroxy-1-oxo-1*H-*indene-2-carbaldehyde, 3-hydroxy-4,5-dimethoxy-1-oxo-1*H*-indene-2-carbaldehyde, 3-hydroxy-5-methoxy-1-oxo-1*H*-indene-2-carbaldehyde, 3-methyl-5-(4-morpholinyl)-1-phenyl-1*H*-pyrazole-4-carbaldehyde, 3-methyl-1-phenyl-5-(1*H*-pyrrol-1-yl)-1*H*-pyrazole-4-carbaldehyde, 3-methyl-1-phenyl-5-(1-pyrrolidinyl)-1*H*-pyrazol-4-carbaldehyde, 3-methyl-1-(2-pyridinyl)-5-(1-pyrrolidinyl)-1*H*-pyrazole-4-carbaldehyde, 3-methyl-1-(2-pyridinyl)-5-(1*H*-pyrrol-1-yl)-1*H*-pyrazole-4-carbaldehyde, 4-methoxy-1-naphthaldehyde, 1*H*-indole-3-carbaldehyde, bis[4-(dimethylamino)-phenyl]methanethione, 2,4-dihydroxybenzaldehyde, 1-methyl-1*H*-indole-3-carbaldehyde and 3,4-dihydroxybenzaldehyde.

4. Agent according to one of Claims 1 to 3, **characterized in that** it comprises the oxime compound of the general formula **A1-A23** and the reactive carbonyl compound of the general formula **B1-B17** in each case in an amount of from 0.01 to 10 per cent by weight.

5. Agent according to one of Claims 1 to 4, **characterized in that** it additionally comprises one or more customary direct dyes from the group of acidic and basic dyes, nitro dyes, azo dyes, anthraquinone dyes and triphenylmethane dyes.

6. Agent according to one of Claims 1 to 5, **characterized in that** it is a hair colorant.

7. Agent according to one of Claims 1 to 6, **characterized in that** it additionally comprises at least one natural or synthetic polymer or modified polymer of natural origin customary for cosmetic compositions, and is in the form of a tinting setting composition or colour setting composition.

8. Method of colouring hair in which a colorant according to one of Claims 1 to 6 is applied to the hair in an amount adequate for the hair colouring, 30 to 120 grams depending on the length of hair, the hair colorant is left to act at 15 to 45 degrees Celsius for 1 to 60 minutes, and the hair is then thoroughly rinsed with water, optionally washed with a shampoo and/or after-treated with a hair-conditioning composition and finally dried.

9. Use of a combination of a) ascorbic acid, b) at least one oxime compound of the formula **A1**-**A23** as in Claim 1 or 2 and c) at least one reactive carbonyl compound of the formula **B1**-**B17** as in Claim 1 or 3 for colouring keratin fibres.

## Patentansprüche

1. Mittel zum Färben keratinhaltiger Fasern, **dadurch gekennzeichnet, dass** es Folgendes umfasst a) Ascorbinsäure, b) mindestens eine Oximverbindung der Formel **A1-A23**; 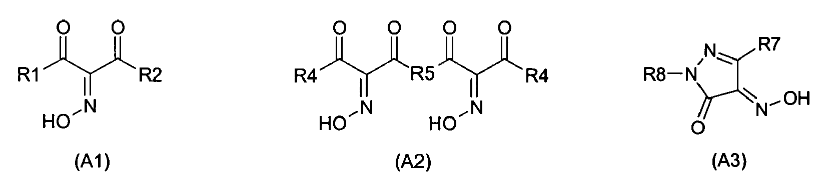 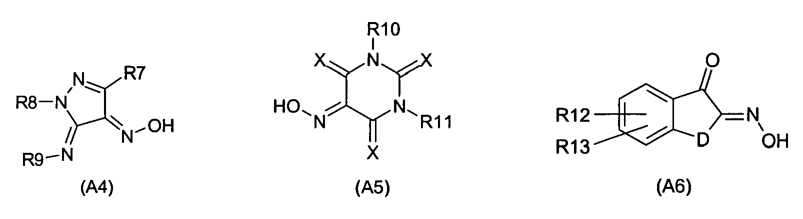 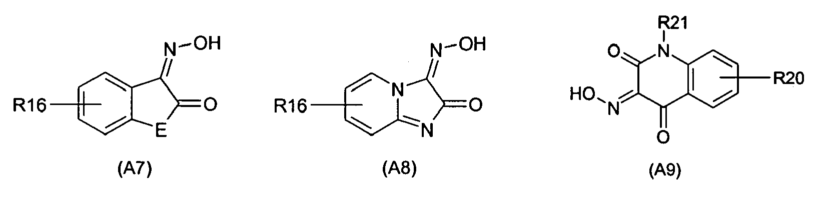 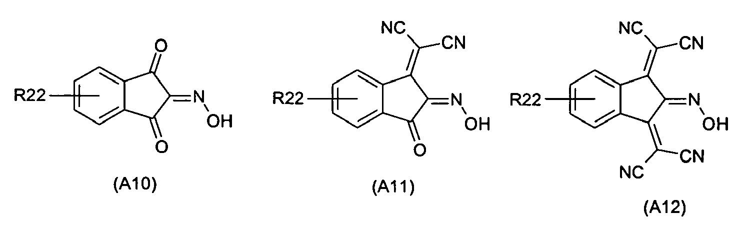 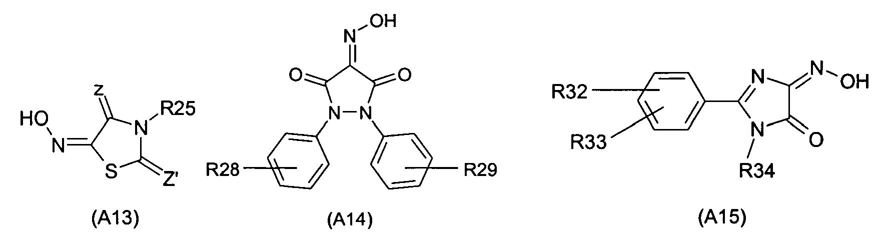 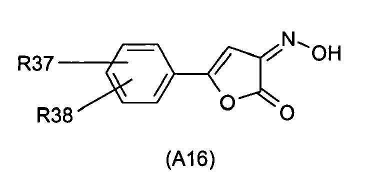 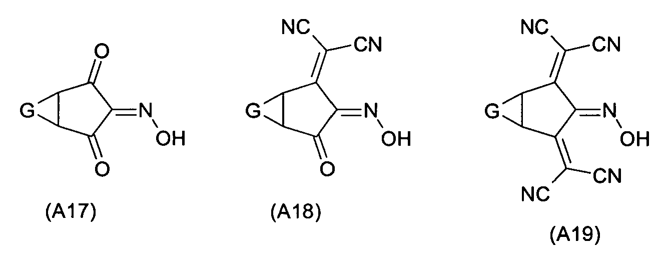 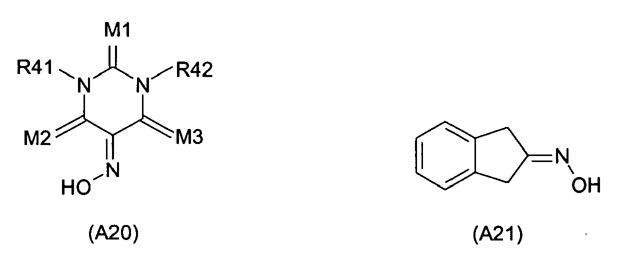 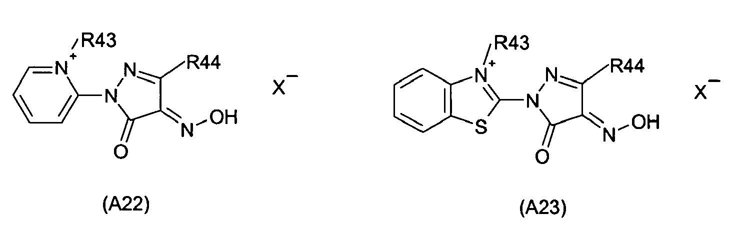 worin
**R1** eine Cyanogruppe, eine substituierte oder unsubstituierte Arylgruppe oder ein substituierter oder unsubstituierter, gesättigter oder ungesättigter Heterocyclus oder eine Gruppe -C-(O)-R3 ist, worin **R3** ein Wasserstoffatom oder eine C₁-C₆-Alkylgruppe ist;
**R2** eine Cyanogruppe, eine substituierte oder unsubstituierte C₁-C₆-Alkylgruppe, eine C₃-C₆-Cycloalkylgruppe, eine substituierte oder unsubstituierte Arylgruppe oder eine substituierte oder unsubstituierte Aminoarylgruppe oder ein substituierter oder unsubstituierter, gesättigter oder ungesättigter Heterocyclus ist;
**R4** eine Cyanogruppe, eine substituierte oder unsubstituierte Arylgruppe oder ein substituierter oder unsubstituierter, gesättigter oder ungesättigter Heterocyclus oder eine Gruppe -C-(O)-R6 ist, worin **R6** ein Wasserstoffatom oder eine C₁-C₆-Alkylgruppe ist;
**R5** eine substituierte oder unsubstituierte C₂-C₆-Alkylgruppe, eine C₃-C₆-Cycloalkylgruppe, eine substituierte oder unsubstituierte Arylgruppe, eine substituierte oder unsubstituierte Aminoarylgruppe, ein substituierter oder unsubstituierter, gesättigter oder ungesättigter Heterocyclus ist;
worin **R7** und **R8** gleich oder verschieden sein können und unabhängig voneinander eine substituierte oder unsubstituierte Arylgruppe, eine Aryl-C₁-C₄-Alkylgruppe, ein substituierter oder unsubstituierter, gesättigter oder ungesättigter Heterocyclus, eine substituierte oder unsubstituierte C₁-C₆-Alkylgruppe, eine Acetyloxygruppe, eine C₃-C₆-Cycloalkylgruppe, eine substituierte oder unsubstituierte Aminoarylgruppe sind, worin **R7** gleichermaßen eine C₁-C₆-Alkoxygruppe sein kann;
**R9** ein Wasserstoffatom oder eine substituierte oder unsubstituierte C₁-C₆-Alkylgruppe ist;
**R10** und **R11** gleich oder verschieden sein können und unabhängig voneinander eine substituierte oder unsubstituierte Arylgruppe, eine Aryl-C₁-C₄-Alkylgruppe, eine C₁-C₆-Alkylgruppe, eine C₃-C₆-Cycloalkylgruppe, eine C₂-C₆-Alkylengruppe oder ein Bicyclus, der über die Radikale R10 und R11 angebunden ist;
**X** ein Sauerstoffatom oder ein Schwefelatom ist;
**D** ein Sauerstoffatom, ein Schwefelatom, eine Sulfoxylgruppe, eine Sulfonylgruppe oder eine Gruppe N-R₁₂ₐ ist, worin **R₁₂ₐ** ein Wasserstoffatom oder eine substituierte oder unsubstituierte C₁-C₆-Alkylgruppe ist;
und **R12** und **R13** unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR14R15 sind, worin **R14** und **R15** unabhängig voneinander ein Wasserstoffatom oder eine C₁-C₆-Alkylgruppe sind;
**E** ein Sauerstoffatom, ein Schwefelatom oder eine Gruppe NR17 ist, worin **R17** ein Wasserstoffatom oder eine substituierte oder unsubstituierte C₁-C₆-Alkylgruppe ist;
**R16** ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR18R19 ist, worin **R18** und **R19** unabhängig voneinander ein Wasserstoffatom oder eine C₁-C₆-Alkylgruppe sind;
**R20** ein Wasserstoffatom, ein Halogenatom, eine Hydroxylgruppe, eine Nitrogruppe, eine C₁-C₆-Alkoxygruppe oder eine substituierte oder unsubstituierte C₁-C₆-Alkylgruppe ist;
**R21** ein Wasserstoffatom oder eine C₁-C₄-Alkylgruppe ist;
**R22** ein Wasserstoffatom, ein Halogenatom, eine Hydroxylgruppe, eine Nitrogruppe, eine C₁-C₆-Alkylgruppe, eine C₁-C₆-Alkoxygruppe, eine Carboxylgruppe, eine Carboxamidgruppe, eine Cyanogruppe oder eine Aminogruppe -NR23R24 ist, worin **R23** und **R24** unabhängig voneinander ein Wasserstoffatom oder eine C₁-C₆-Alkylgruppe sind;
**Z** ein Sauerstoffatom oder eine Gruppe -NR26 ist, worin **R26** ein Wasserstoffatom oder eine C₁-C₆-Alkylgruppe ist;
**Z'** ein Schwefelatom oder eine Gruppe -NR27 ist, worin **R27** ein Wasserstoffatom oder eine C₁-C₆-Alkylgruppe ist;
**R25** ein Wasserstoffatom, eine C₁-C₆-Alkylgruppe oder eine C₁-C₄-Carboxyalkylgruppe ist;
**R28** und **R29** gleich oder verschieden sein können und unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR30R31 sind, worin **R30** und **R31** unabhängig voneinander ein Wasserstoffatom oder eine C₁-C₆-Alkylgruppe sind;
**R32** und **R33** gleich oder verschieden sein können und unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR35R36 sind, worin **R35** und **R36** unabhängig voneinander ein Wasserstoffatom oder eine C₁-C₆-Alkylgruppe sind;
**R34** ein Wasserstoffatom oder eine C₁-C₆-Alkylgruppe ist;
**R37** und **R38** gleich oder verschieden sein können und unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR39R40 sind, worin **R39** und **R40** unabhängig voneinander ein Wasserstoffatom oder eine C₁-C₆-Alkylgruppe sind;
**G** ein kondensierter, substituierter oder unsubstituierter aromatischer oder heteroaromatischer Ring ist, an den zusätzlich ein weiterer aromatischer oder heteroaromatischer Ring kondensiert sein kann;
**R41** und **R42** gleich oder verschieden sein können und unabhängig voneinander ein Wasserstoffatom oder eine substituierte oder unsubstituierte C₁-C₆-Alkylgruppe sind;
**M1** ein Sauerstoffatom, ein Schwefelatom oder eine NH-Gruppe ist;
und **M2** und **M3** gleich oder verschieden sein können und unabhängig voneinander ein Sauerstoffatom oder eine NH-Gruppe sind;
**R43** eine substituierte oder unsubstituierte C₁-C₆-Alkylgruppe ist; **R44** eine substituierte oder unsubstituierte Arylgruppe oder eine Aryl-C₁-C₄-Alkylgruppe, ein substituierter oder unsubstituierter, gesättigter oder ungesättigter Heterocyclus, eine substituierte oder unsubstituierte C₁-C₆-Alkylgruppe, eine Acetyloxygruppe, eine C₃-C₆-Cycloalkylgruppe, eine C₁-C₆-Alkoxygruppe oder eine substituierte oder unsubstituierte Aminoarylgruppe ist; und
**X⁻** ein Gegenion ist;
und c) mindestens eine reaktionsfähige Carbonylverbindung der Formel **B1-B17;** 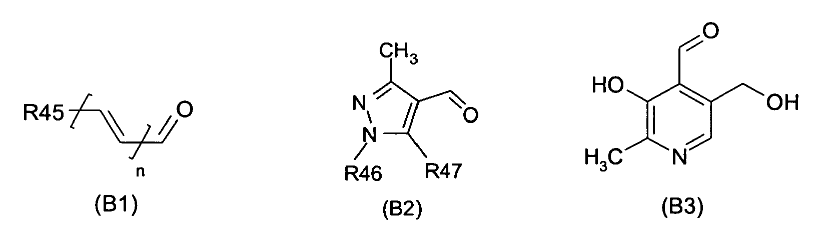 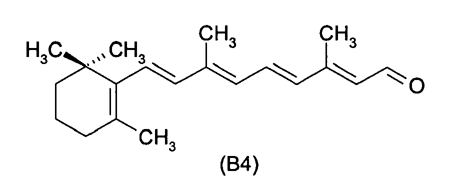 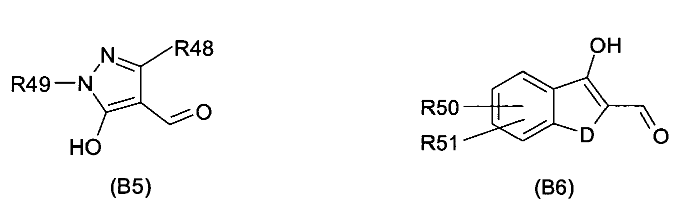 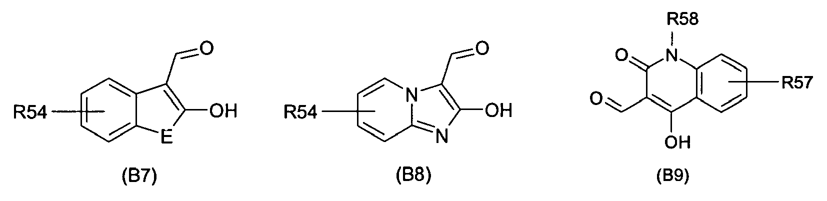 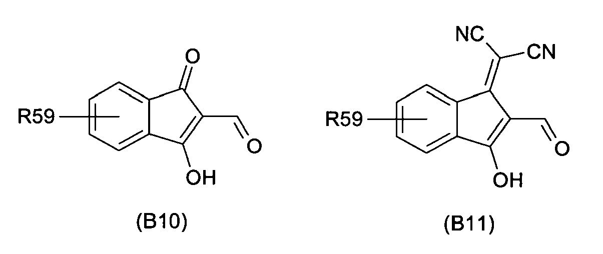 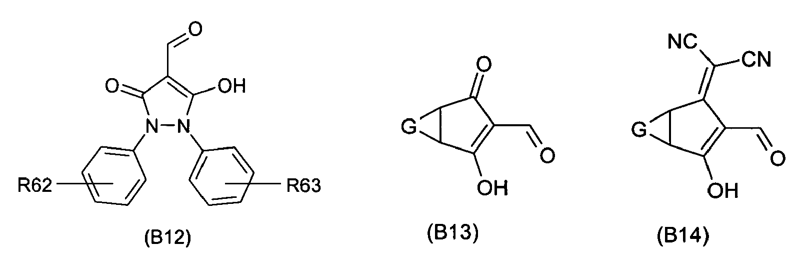 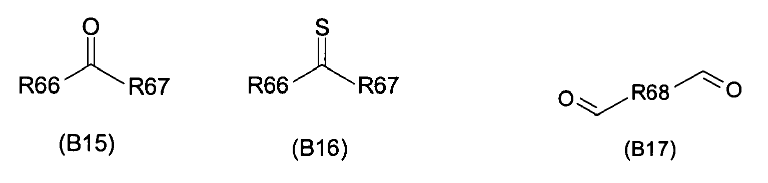 worin
**R45** eine substituierte oder unsubstituierte Arylgruppe ist, an die wahlweise auch ein weiterer aromatischer Ring kondensiert werden kann, ein substituierter oder unsubstituierter, gesättigter oder ungesättigter Heterocyclus;
**n** 0, 1, 2 oder 3 ist;
**R46** eine C₁-C₆-Alkylgruppe, ein substituiertes oder unsubstituiertes Arylradikal oder ein substituierter oder unsubstituierter, gesättigter oder ungesättigter Heterocyclus ist;
**R47** ein Pyrrolradikal, ein Imidazolradikal, ein Pyrazolradikal, ein Indolradikal, ein Pyrrolidinradikal, ein Morpholinradikal, ein Dimethylaminradikal, ein Phenolradikal oder ein Thiophenolradikal ist, wobei diese Radikale in jedem Fall über das Heteroatom an die allgemeine Formel **B2** gebunden sind;
**R48** und **R49** gleich oder verschieden sein können und unabhängig voneinander eine substituierte oder unsubstituierte Arylgruppe, eine Aryl-C₁-C₄-Alkylgruppe, ein substituierter oder unsubstituierter, gesättigter oder ungesättigter Heterocyclus, eine substituierte oder unsubstituierte C₁-C₆-Alkylgruppe, eine Acetyloxygruppe, eine C₃-C₆-Cycloalkylgruppe, eine substituierte oder unsubstituierte Aminoarylgruppe sind, und **R48** gleichermaßen eine C₁-C₆-Alkoxygruppe sein kann;
**D** die vorstehend genannte Bedeutung hat;
**R50** und **R51** gleich oder verschieden sein können und unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR52R53 sind, worin **R52** und **R53** unabhängig voneinander ein Wasserstoffatom oder eine C₁-C₆-Alkylgruppe sind;
**E** die vorstehend genannte Bedeutung hat;
**R54** ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR55R56 ist, worin **R55** und **R56** unabhängig voneinander ein Wasserstoffatom oder eine C₁-C₆-Alkylgruppe sind;
**R57** ein Wasserstoffatom, ein Halogenatom, eine Hydroxylgruppe, eine Nitrogruppe, eine C₁-C₆-Alkoxygruppe oder eine substituierte oder unsubstituierte C₁-C₆-Alkylgruppe ist;
**R58** ein Wasserstoffatom oder eine C₁-C₄-Alkylgruppe ist;
**R59** ein Wasserstoffatom, ein Halogenatom, eine Hydroxylgruppe, eine Nitrogruppe, eine C₁-C₆-Alkylgruppe, eine C₁-C₆-Alkoxygruppe, eine Carboxylgruppe, eine Carboxamidgruppe, eine Cyanogruppe oder eine Aminogruppe -NR60R61 ist, worin **R60** und **R61** unabhängig voneinander ein Wasserstoffatom oder eine C₁-C₆-Alkylgruppe sind;
**R62** und **R63** gleich oder verschieden sein können und unabhängig voneinander ein Wasserstoffatom, ein Fluoratom, ein Chloratom, ein Bromatom, eine Hydroxylgruppe, eine Nitrogruppe, eine C₁-C₆-Alkylgruppe, eine C₁-C₆-Alkoxygruppe, eine Carboxamidgruppe, eine Sulfonamidgruppe, eine Carboxylgruppe, eine C₁-C₄-Acylgruppe, eine Cyanogruppe oder eine Aminogruppe -NR64R65 sind, worin **R64** und **R65** unabhängig voneinander ein Wasserstoffatom oder eine C₁-C₆-Alkylgruppe sind;
worin **G** die vorstehend genannte Bedeutung hat;
**R66** und **R67** gleich oder verschieden sein können und unabhängig voneinander eine C₁-C₆-Alkylgruppe oder eine substituierte oder unsubstituierte Arylgruppe sind; und
**R68** eine substituierte oder unsubstituierte C₁-C₆-Alkylgruppe, eine C₁-C₆-Alkylengruppe oder eine substituierte oder unsubstituierte Arylgruppe ist.

2. Mittel nach Anspruch 1, **dadurch gekennzeichnet, dass** die Oximverbindung der allgemeinen Formel **A1-A23** ausgewählt ist aus Diethyl-2-(hydroxyimino)-malonat, 1*H*-Inden-1,2,3-trion-2-oxim, (2*Z*)-5-(Dimethylamino)-1*H*-inden-1,2,3-trion-2-oxim, (2*Z*)-4,5 -Dimethoxy-1*H*-inden-1,2,3-trion-2-oxim, (2*Z*)-5-Methoxy-1*H*-inden-1,2,3-trion 2-oxim, (2*Z*)-5-Nitro-1*H*-inden-1,2,3-trion-2-oxim, (2*Z*)-4,6-Dichlor-1*H*-inden-1,2,3-trion 2-oxim, (6*Z*)-5*H*-Cyclopenta[*c*]pyridin-5,6,7-trion-6-oxim, (6*Z*)-5*H*-Cyclopenta[*b*]pyridin-5,6,7-trion-6-oxim, (2*E*)-1*H*-Cyclopenta-[*a*]-naphthalin-1,2,3-trion-2-oxim, 1*H*-Cyclopenta[*b*]chinoxalin-1,2,3-trion-2-oxim, (2*Z*)-4-Methyl-4,8b-dihydrocydopenta[*b*]indol-1,2,3(3a*H*)-trion-2-oxim, (5*E)-*4*H-*Cyclopenta[*b*]thiophen-4,5,6-trion-5-oxim, (2*Z*)-5-Methyl-1*H*-inden-1,2,3-trion-2-oxim, (2*E*)-4,6-Dichlor-1*H-*inden-1,2,3-trion-2-oxim, 4,7-Dichlor-1*H*-inden-1,2,3-trion-2-oxim, (2*Z*)-1*H*-Cyclopenta[*b*]chinolin-1,2,3-trion-2-oxim, (2*Z*)-1-Benzothiophen-2,3-dion-2-oxim 1,1-Dioxid, (2*Z*)-1-Benzothiophen-2,3-dion-2-oxim, (2*Z*)-1-Benzofuran-2,3-dion-2-oxim, (3*E*)-Imidazo[1,2-*a*]pyridin-2,3-dion-3-oxim, (3*Z*)-1*H*-Indol-2,3-dion-3-oxim, (3*Z*)-1-Methyl-1*H*-indol-2,3-dion-3-oxim, (3*Z*)-6-Chlor-1-methyl-1*H*-indol-2,3-dion-3-oxim, (3*E*)-7-Chlorimidazo-[1,2-*a*]pyridin-2,3-dion-3-oxim, 1,2-Diphenyl-3,4,5-pyrazolidintrion-4-oxim, 1*H*-Cyclopenta[*b*]naphthalin-1,2,3-trion-2-oxim, 2-[(2*E*)-2-(Hydroxyimino)-3-oxo-2,3-dihydro-1*H*-inden-1-yliden]malononitril, (4*Z*)-3-Methyl-1-phenyl-1*H-*pyrazol-4,5-dion-4-oxim, 1,3-Diethyl-2-thioxodihydro-4,5,6(1*H*)-pyrimidintrion-5-oxim, (5*Z*)-2-Thioxo-1,3-thiazolidin-4,5-dion-5-oxim, (4*Z*)-3-Methyl-1-(4-nitrophenyl)-1*H*-pyrazol-4,5-dion-4-oxim, 1,3-Dihydro-2*H*-inden-2-onoxim, 4,5,6,7-Tetrachlor-1*H*-inden-1,2,3-trion-2-oxim, 2-[(4*E*)-4-(Hydroxyimino)-3-methyl-5-oxo-4,5-dihydro-1*H*-pyrazol-1-yl]-1-methylpyridiniummethylsulfat und 2-[(4*E*)-4-(Hydroxyimino)-3-methyl-5-oxo-4,5-dihydro-1*H*-pyrazol-1-yl]-3-methyl-1,3-benzothiazol-3-iummethylsulfat.

3. Mittel nach Anspruch 1, **dadurch gekennzeichnet, dass** die reaktionsfähige Carbonylverbindung der allgemeinen Formeln **B1-B17** ausgewählt ist aus Benzaldehyd, 4-Methoxybenzaldehyd, 4-Hydroxybenzaldehyd, 3,4-Dimethoxybenzaldehyd, 2,5-Dimethoxybenzaldehyd, 3-Hydroxy-4-methoxybenzaldehyd, 4-Nitrobenzaldehyd, 4-Hydroxy-3-methoxybenzaldehyd, 4-(Dimethylamino)benzaldehyd, (2*E*)-3-[4-(Dimethylamino)phenyl]-2-propenal, 2,4,6-Trihydroxybenzaldehyd, 3-Hydroxy-5-(hydroxymethyl)-2-methylisonicotinaldehyd, all-trans-Retinal, 3,5-Dioxo-1,2-diphenyl-4-pyrazolidincarbaldehyd, (2*E*,4*E*)-5-Phenyl-2,4-pentadienal, Pentandial, 4-(Methylsulfanyl)benzaldehyd, 1-Naphthaldehyd, (2*E*)-3-(4-Nitrophenyl)-2-propenal, 3-Phenoxybenzaldehyd, Terephthalaldehyd, 3-Hydroxy-1-oxo-1*H*-cyclopenta[*a*]-naphthalin-2-carbaldehyd, 3-Hydroxy-1-oxo-1*H*-cyclopenta[*b*]-chinoxalin-2-carbaldehyd, 3-Hydroxy-4-methyl-1-oxo-1,3a,4,8b-tetrahydrocyclopenta[*b*]indol-2-carbaldehyd, 4-Hydroxy-6-oxo-6*H-*Cyclopenta[*b*]thiophen-5-carbaldehyd, 3-Hydroxy-5-nitro-1-oxo-1*H*-inden-2-carbaldehyd, 4,6-Dichlor-3-hydroxy-1-oxo-1*H*-inden-2-carbaldehyd, 7-Hydroxy-5-oxo-5*H*-cyclopenta[*c*]pyridin-6-carbaldehyd, 7-Hydroxy-5-oxo-5*H*-cyclopenta[*b*]pyridin-6-carbaldehyd, 3-hydroxy-1-oxo-1*H*-inden-2-carbaldehyd, 5-(Dimethylamino)-3-hydroxy-1-oxo-1*H*-inden-2-carbaldehyd, 3-Hydroxy-4,5-dimethoxy-1-oxo-1*H*-inden-2-carbaldehyd, 3-Hydroxy-5-methoxy-1-oxo-1*H-*inden-2-carbaldehyd, 3-Methyl-5-(4-morpholinyl)-1-phenyl-1*H*-pyrazol-4-carbaldehyd, 3-Methyl-1-phenyl-5-(1*H*-pyrrol-1-yl)-1*H*-pyrazol-4-carbaldehyd, 3-Methyl-1-phenyl-5-(1-pyrrolidinyl)-1*H*-pyrazol-4-carbaldehyd, 3-Methyl-1-(2-pyridinyl)-5-(1 -pyrrolidinyl)-1*H*-pyrazol-4-carbaldehyd, 3-Methyl-1-(2-pyridinyl)-5-(1*H*-pyrrol-1-yl)- 1*H*-pyrazol-4-carbaldehyd, 4-Methoxy-1-naphthaldehyd, 1*H*-Indol-3-carbaldehyd, Bis[4-(dimethylamino)-phenyl]methanthion, 2,4-Dihydroxybenzaldehyd, 1-Methyl-1*H*-indol-3-carbaldehyd und 3,4-Dihydroxybenzaldehyd.

4. Mittel nach einem der Ansprüche 1 bis 3, **dadurch gekennzeichnet, dass** es die Oximverbindung der allgemeinen Formel **A1-A23** und die reaktionsfähige Carbonylverbindung der allgemeinen Formel **B1-B17** in jedem Fall in einer Menge von 0,01 bis 10 Gewichtsprozent umfasst.

5. Mittel nach einem der Ansprüche 1 bis 4, **dadurch gekennzeichnet, dass** es zusätzlich ein oder mehrere gebräuchliche Direktfarbstoffe aus der Gruppe von sauren und basischen Farbstoffen, Nitrofarbstoffen, Azofarbstoffen, Anthrachinonfarbstoffen und Triphenylmethanfarbstoffen umfasst.

6. Mittel nach einem der Ansprüche 1 bis 5, **dadurch gekennzeichnet, dass** es ein Haarfärbemittel ist.

7. Mittel nach einem der Ansprüche 1 bis 6, **dadurch gekennzeichnet, dass** es zusätzlich mindestens ein natürliches oder synthetisches Polymer oder modifiziertes Polymer natürlicher Herkunft, das für Kosmetikzusammensetzungen gebräuchlich ist, umfasst und in der Form einer Tönungsfixierungszusammensetzung oder Farbfixierungszusammensetzung ist.

8. Verfahren zum Färben von Haar, wobei ein Farbstoff nach einem der Ansprüche 1 bis 6 in einer zu Haarfärbung adäquaten Menge, 30 bis 120 Gramm je nach der Haarlänge, auf das Haar aufgetragen wird, das Haarfärbemittel bei 15 bis 45 Grad Celsius für 1 bis 60 Minuten einwirken gelassen wird und das Haar dann gründlich mit Wasser gespült, wahlweise mit einem Shampoo gewaschen und/oder mit einer Haarkonditionierungszusammensetzung nachbehandelt und schließlich getrocknet wird.

9. Verwendung einer Kombination von a) Ascorbinsäure, b) mindestens einer Oximverbindung der Formel **A1-A23** nach Anspruch 1 oder 2 und c) mindestens einer reaktionsfähigen Carbonylverbindung der Formel **B1-B17** nach Anspruch 1 oder 3 zum Färben von Keratinfasern.

## Revendications

1. Agent pour colorer des fibres contenant de la kératine, **caractérisé en ce qu'**il comprend a) de l'acide ascorbique, b) au moins un composé oxime de formule **A1-A23 ;** 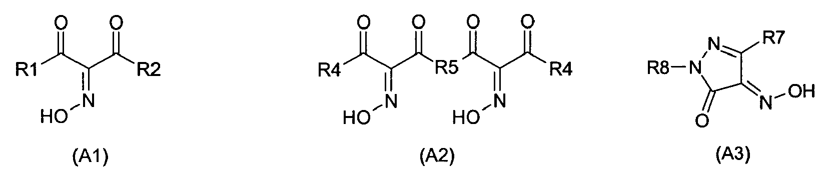 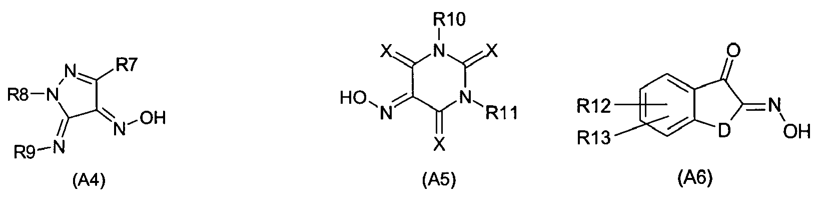 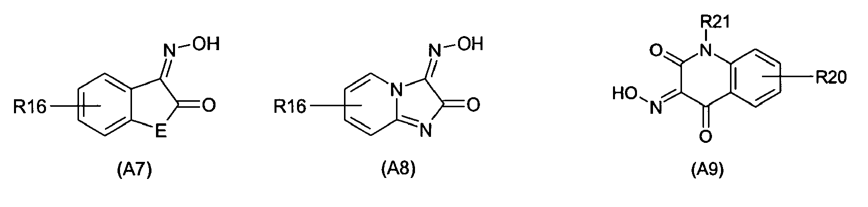 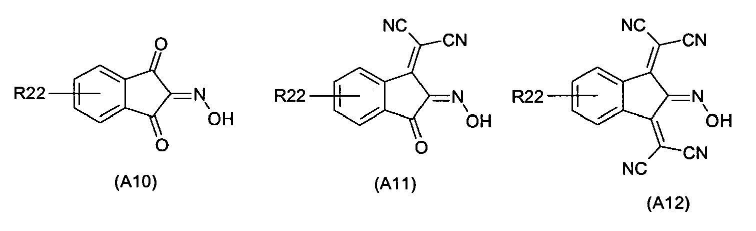 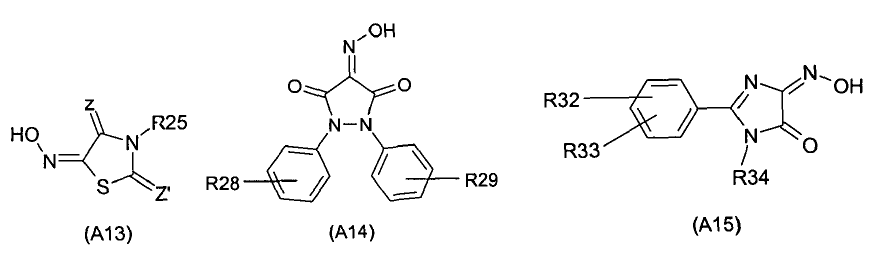 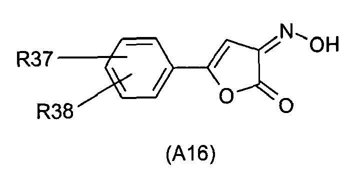 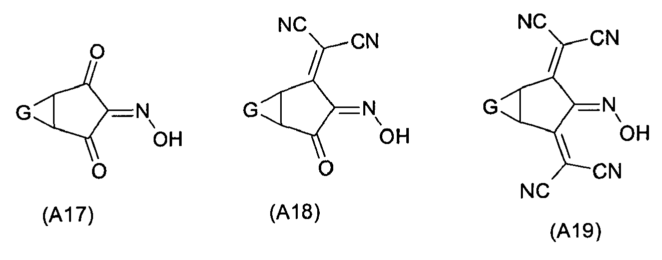 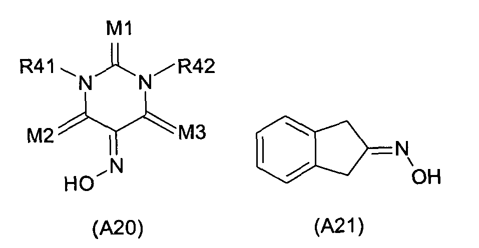 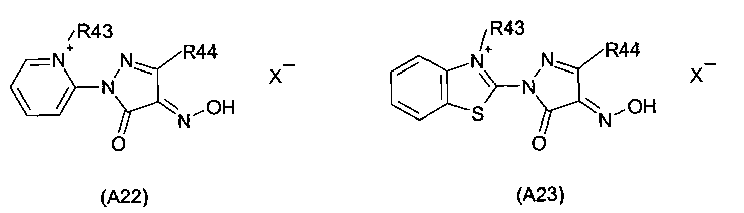 dans laquelle :
**R1** est un groupe cyano, un groupe aryle substitué ou non substitué ou un hétérocycle substitué ou non substitué, saturé ou insaturé ou un groupe -C-(O)-R3, où **R3** est un atome d'hydrogène ou un groupe alkyle en C₁ à C₆ ;
**R2** est un groupe cyano, un groupe alkyle substitué ou non substitué en C₁ à C₆, un groupe cycloalkyle en C₃ à C₆, un groupe aryle substitué ou non substitué ou un groupe aminoaryle substitué ou non substitué ou un hétérocycle substitué ou non substitué, saturé ou insaturé ;
**R4** est un groupe cyano, un groupe aryle substitué ou non substitué, un hétérocycle substitué ou non substitué, saturé ou insaturé ou un groupe -C-(O)-R6, où **R6** est un atome d'hydrogène ou un groupe alkyle en C₁ à C₆ ;
**R5** est un groupe alkyle substitué ou non substitué en C₂ à C₆, un groupe cycloalkyle en C₃ à C₆, un groupe aryle substitué ou non substitué, un groupe aminoaryle substitué ou non substitué, un hétérocycle substitué ou non substitué, saturé ou insaturé ;
dans laquelle **R7** et **R8** peuvent être identiques ou différents et, indépendamment l'un de l'autre, sont un groupe aryle substitué ou non substitué, un groupe aryl-alkyle en C₁ à C₄, un hétérocycle substitué ou non substitué, saturé ou insaturé, un groupe alkyle substitué ou non substitué en C₁ à C₆, un groupe acétyloxy, un groupe cycloalkyle en C₃ à C₆, un groupe aminoaryle substitué ou non substitué, où **R7** peut de façon similaire être un groupe alcoxy en C₁ à C₆ ;
**R9** est un atome d'hydrogène ou un groupe alkyle substitué ou non substitué en C₁ à C₆ ;
**R10** et **R11** peuvent être identiques ou différents et, indépendamment l'un de l'autre, sont un groupe aryle substitué ou non substitué, un groupe aryl-alkyle en C₁ à C₄, un groupe alkyle en C₁ à C₆, un groupe cycloalkyle en C₃ à C₆, un groupe alkylène en C₂ à C₆ ou un bicycle lié via les radicaux R10 et R11 ;
**X** est un atome d'oxygène ou un atome de soufre ;
**D** est un atome d'oxygène, un atome de soufre, un groupe sulfoxyle, un groupe sulfonyle ou un groupe N-R_{12a,} où **R₁₂ₐ** est un atome d'hydrogène ou un groupe alkyle substitué ou non substitué en C₁ à C₆ ;
et **R12** et **R13**, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR14R15, où **R14** et **R15,** indépendamment l'un de l'autre, sont un atome d'hydrogène ou un groupe alkyle en C₁ à C₆ ;
**E** est un atome d'oxygène, un atome de soufre ou un groupe NR17, où **R17** est un atome d'hydrogène ou un groupe alkyle substitué ou non substitué en C₁ à C₆ ;
**R16** est un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino - NR18R19, où **R18** et **R19,** indépendamment l'un de l'autre, sont un atome d'hydrogène ou un groupe alkyle en C₁ à C₆ ;
**R20** est un atome d'hydrogène, un atome d'halogène, un groupe hydroxyle, un groupe nitro, un groupe alcoxy en C₁ à C₆ ou un groupe alkyle substitué ou non substitué en C₁ à C₆ ;
**R21** est un atome d'hydrogène ou un groupe alkyle en C₁ à C₄ ;
**R22** est un atome d'hydrogène, un atome d'halogène, un groupe hydroxyle, un groupe nitro, un groupe alkyle en C₁ à C_{6,} un groupe alcoxy en C₁ à C_{6,} un groupe carboxyle, un groupe carboxamide, un groupe cyano ou un groupe amino -NR23R24, où **R23** et **R24,** indépendamment l'un de l'autre, sont un atome d'hydrogène ou un groupe alkyle en C₁ à C₆;
**Z** est un atome d'oxygène ou un groupe -NR26, où **R26** est un atome d'hydrogène ou un groupe alkyle en C₁ à C₆ ;
**Z'** est un atome de soufre ou un groupe -NR27, où **R27** est un atome d'hydrogène ou un groupe alkyle en C₁ à C₆ ;
**R25** est un atome d'hydrogène, un groupe alkyle en C₁ à C₆ ou un groupe carboxyalkyle en C₁ à C₄ ;
**R28** et **R29** peuvent être identiques ou différents et, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR30R31, où **R30** et **R31,** indépendamment l'un de l'autre, sont un atome d'hydrogène ou un groupe alkyle en C₁ à C₆ ;
**R32** et **R33** peuvent être identiques ou différents et, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR35R36, où **R35** et **R36,** indépendamment l'un de l'autre, sont un atome d'hydrogène ou un groupe alkyle en C₁ à C₆ ;
**R34** est un atome d'hydrogène ou un groupe alkyle en C₁ à C₆ ;
**R37** et **R38** peuvent être identiques ou différents et, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR39R40, où **R39** et **R40,** indépendamment l'un de l'autre, sont un atome d'hydrogène ou un groupe alkyle en C₁ à C₆ ;
**G** est un cycle aromatique ou hétéro-aromatique fusionné, substitué ou non substitué auquel un autre cycle aromatique ou hétéro-aromatique peut en outre être fusionné ;
**R41** et **R42** peuvent être identiques ou différents et, indépendamment l'un de l'autre, sont un atome d'hydrogène ou un groupe alkyle substitué ou non substitué en C₁ à C₆ ;
**M1** est un atome d'oxygène, un atome de soufre ou un groupe NH ;
et **M2** et **M3** peuvent être identiques ou différents et, indépendamment l'un de l'autre, sont un atome d'oxygène ou un groupe NH ;
**R43** est un groupe alkyle substitué ou non substitué en C₁ à C₆ ; **R44** est un groupe aryle substitué ou non substitué ou un groupe aryl-alkyle en C₁ à C₄, un hétérocycle substitué ou non substitué, saturé ou insaturé, un groupe alkyle substitué ou non substitué en C₁ à C₆, un groupe acétyloxy, un groupe cycloalkyle en C₃ à C₆, un groupe alcoxy en C₁ à C₆ ou un groupe aminoaryle substitué ou non substitué ; et
**X⁻** est un contre-ion ;
et c) au moins un composé carbonyle réactif de formule **B1-B17** ; 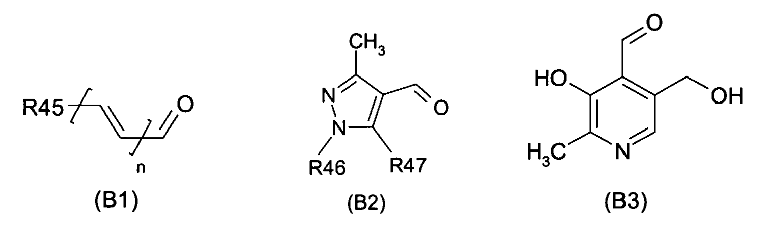 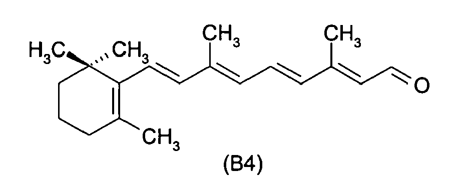 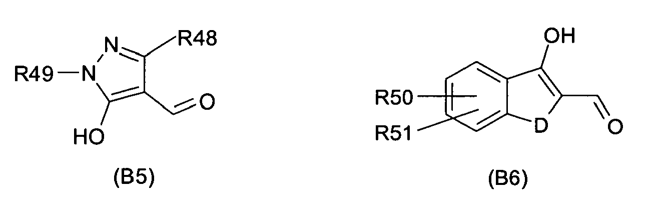 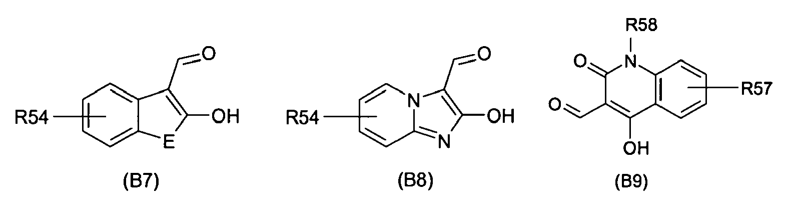 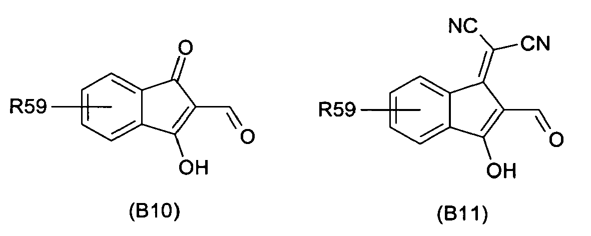 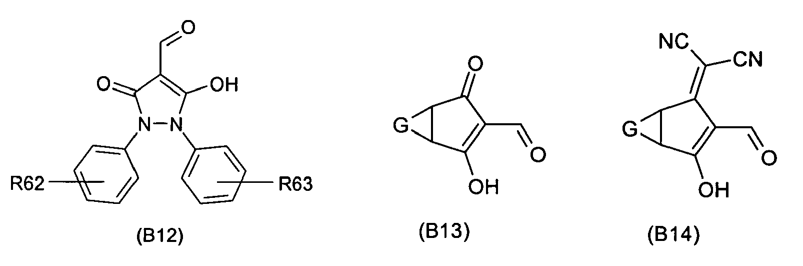 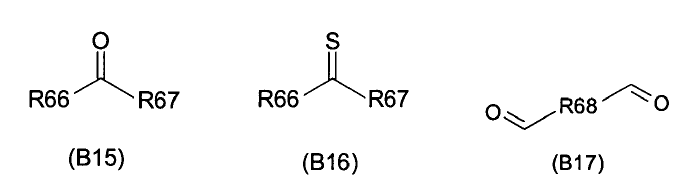 dans laquelle :
**R45** est un groupe aryle substitué ou non substitué auquel un autre cycle aromatique peut facultativement être également fusionné, un hétérocycle substitué ou non substitué, saturé ou insaturé ;
**n** est 0, 1, 2 ou 3 ;
**R46** est un groupe alkyle en C₁ à C₆, un radical aryle substitué ou non substitué ou un hétérocycle substitué ou non substitué, saturé ou insaturé ;
**R47** est un radical pyrrole, un radical imidazole, un radical pyrazole, un radical indole, un radical pyrrolidine, un radical morpholine, un radical diméthylamine, un radical phénol ou un radical thiophénol, où ces radicaux sont dans chaque cas liés à la formule générale **B2** via l'hétéroatome ;
**R48** et **R49** peuvent être identiques ou différents et, indépendamment l'un de l'autre, sont un groupe aryle substitué ou non substitué, un groupe aryl-alkyle en C₁ à C₄, un hétérocycle substitué ou non substitué, saturé ou insaturé, un groupe alkyle substitué ou non substitué en C₁ à C₆, un groupe acétyloxy, un groupe cycloalkyle en C₃ à C₆ ou un groupe aminoaryle substitué ou non substitué, et **R48** peut de façon similaire être un groupe alcoxy en C₁ à C₆ ;
**D** a la signification mentionnée précédemment ;
**R50** et **R51** peuvent être identiques ou différents et, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR52R53, où **R52** et **R53**, indépendamment l'un de l'autre, sont un atome d'hydrogène ou un groupe alkyle en C₁ à C₆ ;
**E** a la signification mentionnée précédemment ;
**R54** est un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino - NR55R56, où **R55** et **R56,** indépendamment l'un de l'autre, sont un atome d'hydrogène ou un groupe alkyle en C₁ à C₆ ;
**R57** est un atome d'hydrogène, un atome d'halogène, un groupe hydroxyle, un groupe nitro, un groupe alcoxy en C₁ à C₆ ou un groupe alkyle substitué ou non substitué en C₁ à C₆ ;
**R58** est un atome d'hydrogène ou un groupe alkyle en C₁ à C₄ ;
**R59** est un atome d'hydrogène, un atome d'halogène, un groupe hydroxyle, un groupe nitro, un groupe alkyle en C₁ à C₆, un groupe alcoxy en C₁ à C₆, un groupe carboxyle, un groupe carboxamide, un groupe cyano ou un groupe amino -NR60R61, où **R60** et **R61**, indépendamment l'un de l'autre, sont un atome d'hydrogène ou un groupe alkyle en C₁ à C₆;
**R62** et **R63** peuvent être identiques ou différents et, indépendamment l'un de l'autre, sont un atome d'hydrogène, un atome de fluor, un atome de chlore, un atome de brome, un groupe hydroxyle, un groupe nitro, un groupe alkyle en C₁ à C₆, un groupe alcoxy en C₁ à C₆, un groupe carboxamide, un groupe sulfonamide, un groupe carboxyle, un groupe acyle en C₁ à C₄, un groupe cyano ou un groupe amino -NR64R65, où **R64** et **R65**, indépendamment l'un de l'autre, sont un atome d'hydrogène ou un groupe alkyle en C₁ à C₆ ;
dans laquelle **G** a la signification mentionnée précédemment ;
**R66** et **R67** peuvent être identiques ou différents et, indépendamment l'un de l'autre, sont un groupe alkyle en C₁ à C₆ ou un groupe aryle substitué ou non substitué ; et
**R68** est un groupe alkyle substitué ou non substitué en C₁ à C₆, un groupe alkylène en C₁ à C₆ ou un groupe aryle substitué ou non substitué.

2. Agent selon la revendication 1, **caractérisé en ce que** le composé oxime de formule générale **A1-A23** est choisi parmi diéthyl 2-(hydroxyimino)malonate, 1*H*-indène-1,2,3-trione 2-oxime, (2*Z*)-5-(diméthylamino)-1*H*-indène-1,2,3-trione 2-oxime, (2*Z*)-4,5-diméthoxy-1*H*-indène-1,2,3-trione 2-oxime, (2*Z*)-5-méthoxy-1*H*-indène-1,2,3-trione 2-oxime, (2*Z*)-5-nitro-1*H*-indène-1,2,3-trione 2-oxime, (2*Z*)-4,6-dichloro-1*H*-indène-1,2,3-trione 2-oxime, (6*Z*)-5*H*-cyclopenta[*c*]pyridine-5,6,7-trione 6-oxime, (6*Z*)-5*H*-cyclopenta[*b*]pyridine-5,6,7-trione 6-oxime, (2*E*)-1*H*-cyclopenta[*a*]-naphtalène-1,2,3-trione 2-oxime, 1*H*-cyclopenta[*b*]quinoxaline-1,2,3-trione 2-oxime, (2*Z*)-4-méthyl-4,8b-dihydrocyclopenta[*b*]indole-1,2,3(3a*H*)-trione 2-oxime, (5*E*)-4*H-*cyclopenta[*b*]thiophène-4,5,6-trione 5-oxime,
(2*Z*)-5-méthyl-1*H*-indène-1,2,3-trione 2-oxime, (2*E*)-4,6-dichloro-1*H*-indène-1,2,3-trione 2-oxime, 4,7-dichloro-1*H*-indène-1,2,3-trione 2-oxime, (2*Z*)-1*H*-cyclopenta[*b*]quinoline-1,2,3-trione 2-oxime, (2*Z*)-1-benzothiophène-2,3-dione 2-oxime 1,1-dioxide, (2*Z*)-1-benzothiophène-2,3-dione 2-oxime, (2*Z*)-1-benzofuran-2,3-dione 2-oxime, (3*E*)-imidazo[1,2-*a*]pyridine-2,3-dione 3-oxime, (3*Z*)-1*H*-indole2,3-dione 3-oxime,
(3*Z*)-1-méthyl-1*H*-indole-2,3-dione 3-oxime, (3*Z*)-6-chloro-1-méthyl-1*H*-indole-2,3-dione 3-oxime, (3*E*)-7-chloro-imidazo[1,2-*a*]pyridine-2,3-dione 3-oxime, 1,2-diphényl-3,4,5-pyrazolidinetrione-4-oxime,
1*H*-cyclopenta[*b*]naphtalène-1,2,3-trione 2-oxime, 2-[(2*E*)-2-(hydroxyimino)-3-oxo-2,3-dihydro-1*H*-indène-1-ylidène]malononitrile,
(4*Z*)-3-méthyl-1-phényl-1*H*-pyrazole-4,5-dione 4-oxime, 1,3-diéthyl-2-thioxodihydro-4,5,6(1*H*)-pyrimidinetrione 5-oxime, (5Z)-2-thioxo-1,3-thiazolidine-4,5-dione 5-oxime, (4*Z*)-3-méthyl-1-(4-nitrophényl)-1*H*-pyrazole-4,5-dione 4-oxime, 1,3-dihydro-2*H*-indèn-2-one oxime,
4,5,6,7-tétrachloro-1*H*-indène-1,2,3-trione 2-oxime, 2-[(4*E*)-4-(hydroxyimino)-3-méthyl-5-oxo-4,5-dihydro-1*H*-pyrazol-1-yl]-1-méthylpyridinium méthylsulfate et 2-[( 4*E*)-4-(hydroxyimino )-3-méthyl-5-oxo-4,5-dihydro-1*H*-pyrazol-1-yl]-3-méthyl-1 ,3-benzothiazol-3-ium méthylsulfate.

3. Agent selon la revendication 1, **caractérisé en ce que** le composé carbonyle réactif de formules générales **B1-B17** est choisi parmi benzaldéhyde, 4-méthoxybenzaldéhyde, 4-hydroxybenzaldéhyde, 3,4-diméthoxybenzaldéhyde, 2,5-diméthoxybenzaldéhyde, 3-hydroxy-4-méthoxybenzaldéhyde, 4-nitrobenzaldéhyde, 4-hydroxy-3-méthoxy-benzaldéhyde, 4-(diméthylamino)benzaldéhyde, (2*E*)-3-[4-(diméthylamino)phényl]-2-propénal, 2,4,6-trihydroxybenzaldéhyde, 3-hydroxy-5-(hydroxyméthyl)-2-méthylisonicotine aldéhyde, rétinal-tout-trans, 3,5-dioxo-1,2-diphényl-4-pyrazolidinecarbaldéhyde, (2*E*,4*E*)-5-phényl-2,4-pentadiénal, pentanedial, 4-(méthylsulfanyl)benzaldéhyde, 1-naphtaldéhyde, (2*E*)-3-(4-nitrophényl)-2-propénal, 3-phénoxybenzaldéhyde, téréphtalaldéhyde, 3-hydroxy-1-oxo-1*H*-cyclopenta[*a*]-naphtalène-2-carbaldéhyde, 3-hydroxy-1-oxo-1*H*-cyclopenta[*b*]-quinoxaline-2-carbaldéhyde, 3-hydroxy-4-méthy1l-1-oxo-1,3a,4,8b-tétrahydrocyclopenta[*b*]indole-2-carbaldéhyde, 4-hydroxy-6-oxo-6H-cyclopenta[*b*]thiophène-5-carbaldéhyde, 3-hydroxy-5-nitro-1-oxo-1*H*-indène-2-carbaldéhyde, 4,6-dichloro-3-hydroxy-1-oxo-1*H*-indène-2-carbaldéhyde, 7-hydroxy-5-oxo-5*H*-cyclopenta[*c*]pyridine-6-carbaldéhyde, 7-hydroxy-5-oxo-5*H-*cyclopenta[*b*]pyridine-6-carbaldéhyde, 3-hydroxy-1-oxo-1*H*-indène-2-carbaldéhyde, 5-(diméthylamino)-3-hydroxy-1-oxo-1*H*-indène-2-carbaldéhyde, 3-hydroxy-4,5-diméthoxy-1-oxo-1*H*-indène-2-carbaldéhyde, 3-hydroxy-5-méthoxy-1-oxo-1*H*-indène-2-carbaldéhyde, 3-méthyl-5-(4-morpholinyl)-1-phényl-1*H*-pyrazole-4-carbaldéhyde, 3-méthyl-1-phényl-5-(1*H*-pyrrol-1-yl)-1*H*-pyrazole-4-carbaldéhyde, 3-méthyl-1-phényl-5-(1-pyrrolidinyl)-1*H*-pyrazol-4-carbaldéhyde, 3-méthyl-1-(2-pyridinyl)-5-(1-pyrrolidinyl)-1*H*-pyrazole-4-carbaldéhyde, 3-méthyl-1-(2-pyridinyl)-5-(1*H*-pyrrol-1-yl)-1*H*-pyrazole-4-carbaldéhyde, 4-méthoxy-1-naphtaldéhyde, 1*H*-indole-3-carbaldéhyde, bis[4-(diméthylamino)-phényl]méthanethione, 2,4-dihydroxybenzaldéhyde, 1-méthyl-1*H-*indole-3-carbaldéhyde et 3,4-dihydroxybenzaldéhyde.

4. Agent selon l'une des revendications 1 à 3, **caractérisé en ce qu'**il comprend le composé oxime de formule générale **A1-A23** et le composé carbonyle réactif de formule générale **B1-B17** dans chaque cas en une quantité allant de 0,01 à 10 pour cent en poids.

5. Agent selon l'une des revendications 1 à 4, **caractérisé en ce qu'**il comprend en outre une ou plusieurs teintures directes d'usage du groupe des teintures acides et basiques, des teintures nitrées, des teintures azoïques, des teintures anthraquinone et des teintures triphénylméthane.

6. Agent selon l'une des revendications 1 à 5, **caractérisé en ce que** c'est une teinture capillaire.

7. Agent selon l'une des revendications 1 à 6, **caractérisé en ce qu'**il comprend en outre au moins un polymère naturel ou synthétique ou polymère modifié d'origine naturelle d'usage pour des compositions cosmétiques, et est sous la forme d'une composition de fixation teintante ou d'une composition de fixation colorante.

8. Procédé de coloration de cheveux dans lequel un colorant selon l'une de revendications 1 à 6 est appliqué sur les cheveux en une quantité adéquate pour la coloration des cheveux, 30 à 120 grammes en fonction de la longueur des cheveux, la teinture capillaire est laissée agir à 15 à 45 degrés Celsius pendant 1 à 60 minutes, et les cheveux sont ensuite soigneusement rincés avec de l'eau, facultativement lavés avec un shampooing et/ou post-traités avec une composition de conditionnement des cheveux et enfin séchés.

9. Utilisation d'une combinaison de a) acide ascorbique, b) au moins un composé oxime de formule **A1-A23** tel que dans la revendication 1 ou 2 et c) au moins un composé carbonyle réactif de formule **B1-B17** tel que dans la revendication 1 ou 3 pour colorer des fibres de kératine.
